# EUROPEAN PATENT SPECIFICATION

(11) **EP 3 619 313 B1**
(45) Date of publication and mention of the grant of the patent: **07.06.2023**
(21) Application number: 19713223.6
(22) Date of filing: 14.02.2019
(51) Int. Cl.: C12N 15/85, C12N 15/86, C12N 15/867, A61K 35/17, A61K 39/00, A61K 48/00, A61P 35/00, C07K 14/725

(54) **TUMOR ENVIRONMENT-SPECIFIC EXPRESSION OF CHIMERIC ANTIGEN RECEPTORS**
TUMOR-UMWELT-SPEZIFISCHE EXPRESSION VON CHIMÄRISCHEN ANTIGEN-REZEPTOREN
EXPRESSION DE RECEPTEURS CHIMERIQUES D'ANTIGENES SPECIFIQUE A UN ENVIRONNEMENT TUMORAL

(30) Priority: 15.02.2018 US 201862631095 P; 23.12.2018 US 201862784501 P
(43) Date of publication of application: 11.03.2020
(73) Proprietor: The National Institute for Biotechnology in the Negev Ltd., 8410501 Beer-Sheva (IL)
(72) Inventor: PORGADOR, Angel, 8533800 Lehavim (IL); GAZIT, Roi, 7079500 Kidron (IL)
(74) Representative: AWA Denmark A/S
(86) International application number: PCT/IL2019/050182
(87) International publication number: WO 2019/159173

(56) References cited:
- JAVAN B. & SHAHBAZI M.: "Hypoxia-inducible tumour-specific promoters as a dual-targeting transcriptional regulation system for cancer gene therapy.", ECANCER, vol. 11, 751, 6 July 2017 (2017-07-06), XP002791514,
- DOTTI G. ET AL.: "Design and development of therapies using chimeric antigen receptor-expressing T cells", IMMUNOL. REV., vol. 257, no. 1, 13 December 2013 (2013-12-13), pages 107-126, XP002791515,
- D. L. Viale ET AL: "Therapeutic Improvement of a Stroma-Targeted CRAd by Incorporating Motives Responsive to the Melanoma Microenvironment", Journal of Investigative Dermatology, vol. 133, no. 11, 1 November 2013 (2013-11-01), pages 2576-2584, XP055719175,
- R. Sakemura ET AL: "A Tet-On Inducible System for Controlling CD19-Chimeric Antigen Receptor Expression upon Drug Administration", CANCER IMMUNOLOGY RESEARCH, vol. 4, no. 8, 21 June 2016 (2016-06-21), pages 658-668, XP055513121, US ISSN: 2326-6066, DOI: 10.1158/2326-6066.CIR-16-0043
- JAVAN B & SHAHBAZI M ED - JAVAN B & SHAHBAZI M: "Hypoxia-inducible tumour-specific promoters as a dual-targeting transcriptional regulation system for cancer gene therapy", ECANCER,, vol. 11, 6 July 2017 (2017-07-06), XP002791514, DOI: 10.3332/ECANCER.2017.751
- GIANPIETRO DOTTI ET AL: "Design and development of therapies using chimeric antigen receptor-expressing T cells", IMMUNOLOGICAL REVIEWS, vol. 257, no. 1, 13 December 2013 (2013-12-13), pages 107-126, XP055552726, US ISSN: 0105-2896, DOI: 10.1111/imr.12131

## Description

### FIELD OF INVENTION

The present disclosure generally relates to the field of chimeric antigen receptor (CAR) expression, specifically to tumor tissue specific CAR expression.

### BACKGROUND

Harnessing the immune system to eradicate cancer has proved highly efficient in recent years.

An example is engineered immune cells such as Chimeric-Antigen-Receptor T-cells (CAR-T), which have been approved by the FDA for the treatment of various cancers after outstanding results were shown regarding the ability to eradicate malignancies that had no other efficient treatment.

However, although CAR expressing immune cells can reach tumors and metastasis throughout a patient's body, the specificity of the engineered receptor does not allow fully distinguishing between tumor cells and normal cells, with a few exceptions, since the tumor often does not have an absolutely unique antigen that is not expressed by some normal cells in the body. As a result, several CAR treatments caused toxic immune response, similar to GVHD, and even death that resulted from the CAR treatment during clinical trials.

Attempts to achieve non-constitutive expression of CAR within engineered immune cells have been made. An example includes applying an "ON-OFF switch" within the CAR expression vector, by utilizing a promoter activated only in the presence of an exogenously provided molecule (such as tetracycline/doxycycline). Albeit allowing turning off the CAR expression in case adverse symptoms is pronounced, this approach also turns off the positive activity of the CAR T-cells against the tumor cells, and thus terminates a potent CAR treatment.

There thus remains an unmet need for controlled CAR expression that reduces the risks of its life-threatening "side-effect", while allowing effective elimination of tumors

Constructs to control the expression of CARs were disclosed, inter alia, in:
Javan (2017); eCancer 11:751
Dotti (2013); Immunol. Rev. 257(1):107-126
Viale (2013); J. Invest. Derm. 133(11):2576-2584
Sakemura (2016); Cancer Immunol. Res. 4(8):658-668

### SUMMARY

According to the present invention there is provided Tumor Micro-Environment (TME) responsive expression vectors according to appended claims 1-7 and immune effector cells according to appended claims 8-9.

The technical disclosure set out below may in some respects go beyond the scope of the invention, which is defined by the appended claims. Elements of the disclosure which do not fall within the scope of the claims are provided for information, namely to place the actual invention claimed in a broader technical context.

Disclosed herein is a novel platform for regulation of effector gene expression under the control of tumor-microenvironment-responsive promoters, optionally in conjunction with a tet-response circuit.

The platform includes a tumor environment (TME) responsive expression vector including a nucleic acid sequence encoding a synthetic promoter comprising one or more TME dependent promoter response element; conjugated to effector-genes such as, but not limited to, nucleic acid sequence encoding an effector gene (e.g. CAR).

Advantageously, the TME responsive vector is designed such that binding of two or more factors, present in the TME, to the promoter response element, either directly or indirectly, induces expression by the promoter. In the absence of TME factors binding, the promoter expression is downregulated autonomously within each of the engineered cells. This advantageously ensures that minimal to no expression of effector-mechanisms, such as CAR, is found in tissue environments different from that of the tumor; whereas in the tumor environment, the expression of effector mechanisms, such as CAR, is upregulated and directing activities against the tumor while sparing normal tissues.

The expression vector include more than one TME dependent promoter response element. This may serve to ensure that the highest expression level is solely obtained where the specific combination of TME factors is found.

Advantageously, we may optionally further replace/change the synthetic promoter for a custom-made promoter, such that the one or more TME dependent promoter response elements, configured to activate the promoter, will fit the actual TME signature of a specific patient or patient group, thus ensuring an uttermost specific and efficient response.

In a first aspect, there is provided a tumor microenvironment (TME) responsive expression vector comprising a nucleic acid sequence encoding a chimeric antigen receptor (CAR) operably linked to a synthetic promoter, said promoter comprising two or more different TME dependent promoter response elements (PRE); wherein said at least two PRE's are an interferon-gamma-(IFN-γ) PRE and an NEκB PRE, and wherein the IFN-γ promoter response element comprises the nucleic acid sequence set forth in SEQ ID NO: 1, and wherein the NEκB PRE comprises the nucleic acid sequence set forth in SEQ ID NO: 2 ("K") ; wherein said TME responsive expression vector is so designed, that the presence of IFN-γ and TNFα in the TME, when binding to interferon-gamma-(IFN-y) PRE and NEκB PRE, induces a higher expression level of CAR than when only IFN-γ or TNFα are pre-sent in the TME, and minimal no or low expression of CAR occurs in the absence of IFN-γ and TNFα, so that the CAR is upregulated and directing activities against the tumor while sparing normal tissue.

According to some embodiments, the CAR is a chimeric antigen T-cell receptor (CAR-T), a chimeric antigen Natural Killer (NK) cell receptor (CAR-NK), a chimeric innate receptor, other immune-effectors including, but not limited to, cytokines, chemokines, chemokine-receptors, proteases, micro-RNAs, or combinations thereof.

According to the invention, the promoter response element comprises an interferon-gamma (IFN-γ) response element, a Nuclear Factor kappa-B (NF-κB) response element and optionally one or more response elements selected from the list consisting of, but not limited to: a hypoxia response element, an IL-6 response elements, a Heat shock protein 70 (HSP-70) response element, an IL-1 response element, an IL-4 response elements, an IL-6 response elements, an IL-8 response element, an IL-10 response element, an IL-11 response element, an IL-12 response element, an IL-15 response element, an IL-18 response element, an IL-17 response element, an IL-21 response element, an IL-35 response element, a TGF-beta response element, a GM-CSF response element, a Hepatic Growth Factor (HGF) response element, an Aryl Hydrogen Receptor (AhR) response element, a PGE2 response element or any other suitable TME factor response element or combinations thereof.

According to some embodiments, the promoter response element may be inserted into the synthetic promoter in a sense (5' to 3') or anti-sense (3' to 5') direction.

The promoter response element comprises at least a nucleic acid selected from the group consisting of: GGGAATTTCC set forth in SEQ ID NO. 2, and in some embodiments may further comprise a nucleic acid selected from the group consisting of TTCCGGGAA set forth in SEQ ID NO. 1, GACCTTGAGTACGTGCGTCTCTGCACGTATG set forth in SEQ ID NO. 3, GCGCTTCCTGACAGTGACGCGAGCCG set forth in SEQ ID NO. 4, GCGCTTCCTGACAGTGACGCGAGCCG, or any combination thereof.

According to some embodiments, the promoter response element further comprises a nucleic acid selected from the group consisting of: 
TACAGGGACAGCAGAGATCCAGTTTGGACTAGCCCGGTCGCACTAGTTCT
AGAACTTCCCGGAAATAGGGTGGGCAAGTATTTCCGGGAAATTCTAGAGG
GAGTTCCCGGGGACTTTCCGGGGATTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 22,
 
TACAGGGACAGCAGAGATCCAGTTTGGACTAGCCCGGTCGCACTAGTTCT
AGAACTTCCCGGAAGTAGGGTGGGCAAGTACTTCCCGGAAGTTCTAGAGG
AAATTTTCGGGGACTTTCCGGGGGTTCTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 23,
 
TACAGGGACAGCAGAGATCCAGTTTGGACTAGCCCGGTCGCACTAGTTCT
AGAACTTCCCGGAAGTAGGGTGGGCAAGTACTTCCGGGAAATTCTAGAGG
GAGTTCTCGGGGACTTTCCGGGAATTTTCTCTAGATATTAAGGTGACGCGT
 
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 24,
 
TACAGGGACAGCAGAGATCCAGTTTGGACTAGCCCGGTCGCACTAGTTCT
AGAACTTCCCGGAAGTAGGGTGGGCAAGTATTTCCCGGAAGTTCTAGAGG
AAGTTCTCGGGGACTTTCCGGAGATTCTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 25,
 
TACAGGGACAGCAGAGATCCAGTTTGGACTAGCCCGGTCGCACTAGTTCT
AGAACTTCCGGGAAATAGGGTGGGCAAGTACTTCCGGGAAATTCTAGAGG
GGATTTCCGGGGACTTTCCGGAGGTTCTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 26,
 
TACAGGGACAGCAGAGATCCAGTTTGGACTAGCCCGGTCGCACTAGTTCT
AGAACTTCCGGGAAATAGGGTGGGCAAGTACTTCCGGGAAGTTCTAGAGG
AGGTTTTCGGGGACTTTCCGGAGGTTTCCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 27,
 
TACAGGGACAGCAGAGATCCAGTTTGGACTAGCCCGGTCGCACTAGTTCT
AGAACTTCCGGGAAGTAGGGTGGGCAAGTATTTCCCGGAAGTTCTAGAGG
GGGTTTTCGGGGACTTTCCGGAGGTTTCCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 28,
 
TACAGGGACAGCAGAGATCCAGTTTGGACTAGCCCGGTCGCACTAGTTCT
AGAACTTCCGGGAAGTAGGGTGGGCAAGTATTTCCGGGAAATTCTAGAGG
GAGTTCTCGGGGACTTTCCGGGGATTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 29,
 
TACAGGGACAGCAGAGATCCAGTTTGGACTAGCCCGGTCGCACTAGTTCT
AGAATTTCCCGGAAATAGGGTGGGCAAGTACTTCCGGGAAATTCTAGAGG
GGGTTTCCGGGGACTTTCCGGGGGTTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 30,
 
TACAGGGACAGCAGAGATCCAGTTTGGACTAGCCCGGTCGCACTAGTTCT
AGAATTTCCCGGAAATAGGGTGGGCAAGTACTTCCGGGAAATTCTAGAGG
GGGTTTCCGGGGACTTTCCGGGGGTTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 31,
 
TACAGGGACAGCAGAGATCCAGTTTGGACTAGCCCGGTCGCACTAGTTCT
AGAATTTCCCGGAAATAGGGTGGGCAAGTATTTCCGGGAAATTCTAGAGG
AGGTTCTCGGGGACTTTCCGGGAGTTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 32,
 
TACAGGGACAGCAGAGATCCAGTTTGGACTAGCCCGGTCGCACTAGTTCT
AGAATTTCCCGGAAATAGGGTGGGCAAGTATTTCCGGGAAATTCTAGAGG
AGGTTTTCGGGGACTTTCCGGGAGTTTCCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 33,
 
TACAGGGACAGCAGAGATCCAGTTTGGACTAGCCCGGTCGCACTAGTTCT
AGAATTTCCCGGAAATAGGGTGGGCAAGTATTTCCGGGAAGTTCTAGAGG
AAGTTTTCGGGGACTTTCCGGGAATTTTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 34,
 
TACAGGGACAGCAGAGATCCAGTTTGGACTAGCCCGGTCGCACTAGTTCT
AGAATTTCCGGGAAATAGGGTGGGCAAGTATTTCCCGGAAGTTCTAGAGG
AGATTCTCGGGGACTTTCCGGGGGTTCTCTCTAGATATTAAGGTGACGCGT
GTGGCCTCGAACACCGAGCGACCCTGCAGCGACCCGCTTAAAAGCGGCCG
 
CCATGGGCCGCCATGGCCTCCTCCGAGGACGTCATCAAGGAGTTCATGC
>set forth in SEQ ID NO: 35,
 
TACAGGGACAGCAGAGATCCAGTTTGGACTAGCCCGGTCGCACTAGTTCT
AGAATTTCCGGGAAGTAGGGTGGGCAAGTACTTCCGGGAAATTCTAGAGG
GGGTTTCCGGGGACTTTCCGGAGATTCTCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 36,
 
TACAGGGACAGCAGAGATCCAGTTTGGACTAGCCCGGTCGCACTAGTTCT
AGAATTTCCGGGAAATAGGGTGGGCAAGTATTTCCCGGAAGTTCTAGAGG
GGGTTTTCGGGGACTTTCCGGAAATTTTCTCTAGATATTAAGGTGACGCGT
GTGGCCTCGAACACCGAGCGACCCTGCAGCGACCCGCTTAAAAGCGGCCG
CCATGGGCCCCATGGCCTCCTCCGAGGACGTCATCAAGGAGTTCATGC >set
forth in SEQ ID NO: 37,
 
TACAGGGACAGCAGAGATCCAGTTTGGACTAGCCCGGTCGCACTAGTTCT
AGAACTTCCCGGAAATAGGGTGGGCAAGTATTTCCCGGAAGTTCTAGAGG
AGGTTTTCGGGGACTTTCCGGGATTCCCTCTAGATATTAAGGTGACGCGTG
TGGCCTCGAACACCGAGCGACCCTGCAGCGACCCGCTTAAAAGCGGCCGC
CATGGGCCGCCATGGCCTCCTCCGAGGACGTCATCAAGGAGTTCATGC >set
forth in SEQ ID NO: 38,
 
TACAGGGACAGCAGAGATCCAGTTTGGACTAGCCCGGTCGCACTAGTTCT
AGTTCCGGGAAGTGGTGGGCAATATTTCCCGGAAGTTTAGAGGAAGTTT
TCGGGGACTTCCGGAAATTCCCTCTAGATATTAAGGTGACGCGTGTGGCC
TCGAACACCGAGCGACCCTGCAGCGACCCGCTTAAAAGCGGCCGCCATGG
GCCGCCATGGCCTCCTCCGAGGACGTCATCAAGGAGTTCATGC >set forth in
SEQ ID NO: 39,
 
TACAGGGACAGCAGAGATCCAGTTTGGACTAGCCCGGTCGCACTAGTTCT
AGAACTTCTCGGAAATAGGGTGGGCAAGTACTGTGGCCTCGAACACCGAG
CGACCCTGCAGCGACCCGCTTAAAAGCGGCCGCCATGGGCCGCCATGGCC
TCCTCCGAGGACGTCATCAAGGAGTTCATGCAGACCAAGTCTCTGCTACC
>set forth in SEQ ID NO: 40
 or combinations thereof. Each possibility is a separate embodiment.

According to the invention, the promoter response element comprises a nucleic acid selected from the nucleic acid sequences set forth in SEQ ID Nos 2.

According to some embodiments, the promoter response element comprises a nucleic acid selected from the nucleic acid sequences set forth in SEQ ID Nos 22-40 or any combination thereof. Each possibility is a separate embodiment.

According to some embodiments, the promoter response element comprises the nucleic acid sequence: 
TACAGGGACAGCAGAGATCCAGTTTGGACTAGCCCGGTCGCACTAGTTCT
AGAAYTTCCSGGAARTAGGGTGGGCAAGTAYTTCCSGGAARTTCTAGAGG
RRRTTYYCGGGGACTTTCCGGRRRTTYYCTCTAGATATTAAGGTGACGCGT
GTGGCCTCGAACACCGAGCGACCCTGCAGCGACCCGCTTAAAAGCGGCCG
CCATGGGCCGCCATGGCCTCCTCCGAGGACGTCATCAAGGAGTTCATGC
>set forth in SEQ ID NO: 41,
 wherein Y=C or T, S= C or G and R= A or G.

According to some embodiments, the one or more TME factor comprises tumor necrosis factor alpha (TNF-α), IFN-γ, IL-6, HSP-70 or any combination thereof.

According to some embodiments, the synthetic promoter comprises additional nucleotides flanking the promoter response element and or spacing between promoter response elements.

The promoter response element comprises IFN-γ and NF-κB, and in further embodiments one or more response element of any of hypoxia protein, HSP-70, IL-1, IL-4, IL-6, IL-8 IL-10 response element, an IL-11 response element, an IL-12 response element, an IL-15 response element, an IL-18 response element, an IL-17 response element, an IL-21 response element, a TGF-beta response element, a GM-CSF response element, a Hepatic Growth Factor (HGF) response element, an Aryl Hydrogen Receptor (AhR) response element, a PGE2 response element (sense or anti-sense), which has been modified on one or more positions.

According to some embodiments, the modification generates a sequence with increased TME factor binding vis-à-vis the native sequence.

As a non-limiting example, the synthetic promoter may include response elements of hypoxia protein (or other TME factor response element) as derived from various hypoxia dependent target genes (LDHA/EPO/VEGF), such as the shared part of the HBS sequence of the LDHA/EPO/VEGF and/or the HAS sequence of EPO gene, as well as a linker of about 6-9 nucleotides which is not found in the target genes. This sequence is referred to as a "basic hypoxia promoter response element (PRE)", as set forth in SEQ ID NO. 42 outlined below.

>SEQ ID NO. 42:
ACGTGGCTGGAGTCACAGTCCTCTTACGTGGCTGGAGTCACAGTCCTCTT
HBS linker1 HAS linker2 HBS linker1 HBS linker2

According to some embodiments, the basic TME factor promoter (here basic hypoxia PRE) may be added at the 3' at the 5' or in the middle of the synthetic promoter sequence. According to some embodiments, the basic TME factor PRE may be inserted in a 5'-3' orientation or in a flipped 3'-5' orientation. According to some embodiments, the synthetic promoter may include a modified version of the basic TME factor PRE. A non-limiting example, of a modified basic hypoxia PRE is set forth in SEQ ID 43: 
>RCGTGSCTGGAGTMACAGTCCTCTTRCGTGSCTGGAGTMACAGTC
CTCTT,
 wherein R= A or G, S= C or G and M = A or C.

According to some embodiments, the modified TME factor PRE is the synthetic PRE that induce the less leakiness and the highest response to hypoxia stimulation.

A non-limiting example for a modified IFN-γ PRE is set forth in SEQ ID 44:
> ACTTCCSGGAARTAGGGTGGGCAAGTACTTCCSGGAART, wherein R= A or G and S= C or G.

A non-limiting example for a modified NF-κB PRE is set forth in SEQ ID 45:
>GGGGGTTTYCGGGGACTTTCCGGRRRTTTT wherein R= A or G andY = C or T.

The promoter response element comprises two or more promoter response elements; and wherein binding of TME factors to the two or more TME dependent promoter response elements induces a higher expression level of effector-genes than binding to a single TME dependent promoter response element.

According to some embodiments, the TME responsive expression vector further comprises an externally inducible promoter and a trans-activator. According to some embodiments, the synthetic promoter drives expression of the trans-activator and the externally inducible promoter drives expression of the effector-genes. According to some embodiments, the combined presence of the inducer and the TME factor induces expression of effector-genes. According to some embodiments, in the presence of the external inducer and in the absence of TME factor, essentially no effector-genes expression is detected. According to some embodiments, when the TME factor binds the promoter response element in the absence of the external inducer, essentially no effector-genes expression is detected.

According to some embodiments, the inducible promoter is a Tet-Response-Element promoter, and the external inducer is doxycycline and/or tetracycline.

According to some embodiments, the effector-gene may be, but is not limited to a CAR that comprises an antigen binding domain, a transmembrane domain, and an intracellular domain comprising a costimulatory domain and/or a primary signaling domain, wherein said antigen binding domain binds to a tumor antigen.

According to some embodiments, the CAR may be selected from the group consisting of CAR, a chemokine receptor, a cytokine receptor, a protein (or functional RNA) that enhances penetration of the immune effector cell in to the tumor, such as, but not limited to proteases of the MMP8/9, a miRNA that suppresses immuneinhibitors, such as, but not limited to PD1 and/or CTLA4, a cytokine that brings about immune-cell retention within the tumor, such as, but not limited to CXCL9/10 and/or CRCR3 ligands or any other suitable effector gene a TME which specific expression profile is desired.

According to some embodiments, the vector is selected from a DNA vector, a plasmid, a lentivirus vector, an adenoviral vector, a retrovirus vector, or other vectors for introduction of the synthetic construct into immune cells.

According to some embodiments, the TME responsive expression vector further comprises effector-genes encoding a protein (or functional RNA) that enhance penetration of the immune effector cell in to the tumor, such as, but not limited to, proteases of the MMP8/9.

According to some embodiments, the TME responsive expression vector further comprises one or more effector-genes encoding miRNAs that suppress immuneinhibitors, such as, but not limited to, PD1 and/or CTLA4, within the tumor.

According to some embodiments, the TME responsive expression vector further comprises one or more effector-genes encoding cytokines bringing about immune-cell retention within the tumor, such as, but not limited to, CXCL9/10 and/or CRCR3 ligands, thereby generating an autocrine loop.

According to some embodiments, there is provided an immune effector cell comprising the vector comprising a nucleic acid sequence encoding a synthetic promoter comprising one or more TME dependent promoter response element; and a nucleic acid sequence encoding a chimeric antigen receptor, as essentially described herein. According to some embodiments, the TME responsive vector is designed, such that binding of one or more factors present in the TME to the promoter response element directly or indirectly induces expression of the effector genes, and wherein, in the absence of TME factor binding to the promoter response element, essentially no or low/residual chimeric antigen receptor is expressed.

According to some embodiments, the immune effector cell is suitable for use as a medicament. According to some embodiments, the immune effector cell is suitable for treating a tumor of a patient in need thereof. According to some embodiments, the tumor is a solid tumor. According to some embodiments, the solid tumor is a sarcoma, a carcinomas or a lymphoma. According to some embodiments, the solid tumor is a lung tumor, melanoma, colon cancer, breast tumor or a brain tumor.

Disclosed herein is a method for treating cancer in a patient in need thereof, the method comprising administering immune cells comprising the expression vector as essentially described herein.

Disclosed herein is a method for screening a patient for determination of an optimal synthetic promoter, the method comprising obtaining a biopsy of a patient's tumor, determining the expression levels of one or more TME factors in the biopsy; and selecting a TME responsive expression vector having a TME dependent promoter response element matching the determined expression level of the one or more TME factors in the biopsy.

Disclosed herein is a method for screening a biopsy for determining an optimal synthetic promoter, the method comprising determining the expression level of one or more TME factors in the biopsy; and selecting a TME responsive expression vector having a TME dependent promoter response element matching the determined expression level of the one or more TME factors in the biopsy.

The TME may be expressed by the tumor cells and/or by non-tumor TME cells.

The method further comprises introducing the selected TME responsive expression vector into immune cells.

The immune effector cell or cell population may include, but is not limited to, T-cells and/or Natural-Killer (NK) cells.

The immune effector cell or cell population is autologous to the patient.

The immune effector cell/cell population is isolated from the patient prior to the treatment.

The method further comprises administering the immune effector cell or cell population to the patient.

Certain embodiments of the present disclosure may include some, all, or none of the above advantages. One or more technical advantages may be readily apparent to those skilled in the art from the figures, descriptions and claims included herein. Moreover, while specific advantages have been enumerated above, various embodiments may include all, some or none of the enumerated advantages.

In addition to the exemplary aspects and embodiments described above, further aspects and embodiments will become apparent by reference to the figures and by study of the following detailed descriptions.

### BRIEF DESCRIPTION OF THE FIGURES

The invention will now be described in relation to certain examples and embodiments with reference to the following illustrative figures.
**FIG. 1** schematically illustrates an expression construct comprising a synthetic promoter directly controlling reporter- and/or CAR gene expression;
**FIG. 2** schematically illustrates an expression construct comprising a synthetic promoter indirectly controlling reporter- and/or CAR gene expression;
**FIG. 3** is an illustrative flowchart of a method for screening a promoters-library for Tumor Micro-Environment (TME) providing an optimal reporter- and/or CAR gene expression;
**FIG. 4A** shows a representative histogram depicting GFP-intensity (upper panel) and percentage of GFP-positive cells (lower panel) in 293T HEK cells transduced with the expression lentiviral construct of FIG. 2 (w. GFP reporter), including the indicated TME responsive elements (G and K), in the presence and absence of TME factor and/or doxycycline;
**FIG. 4B** shows a representative FACS plot of representative replicates used for Figure 3A.
**FIG. 5** shows a representative FACS plot gating GFP-positive 293T HEK cells transfected with the expression construct of FIG. 2 (w. GFP reporter), including the indicated TME responsive elements (G and K), in the presence and absence of TME factor and/or doxycycline;
**FIG. 6** shows representative histograms quantifying the reporter intensity within the GFP-positive 293T HEK cells transfected with the expression construct of FIG. 2 (w. GFP reporter), including the indicated TME responsive elements (G and K), in the presence and absence of TME factor and/or doxycycline;
**FIG. 7** shows a representative histogram quantifying the reporter intensity within the GFP-positive 293T HEK cells transfected with the expression construct of FIG. 2 (w. GFP reporter), including the indicated TME responsive elements (G, K, J and H), in the presence and absence of TME factor and/or doxycycline;
**FIG. 8** shows exemplary FACS sorting results used to identify and sort for cells having a desired, optimal reporter gene expression profile.

### DETAILED DESCRIPTION

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

### Definitions

Unless defined otherwise, all technical and scientific terms used herein have the same meaning as commonly understood by one of ordinary skill in the art to which the invention pertains.

The term "a" and "an" refers to one or to more than one (i.e., to at least one) of the grammatical object of the article. By way of example, "an element" means one element or more than one element.

The term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.

The term "Chimeric Antigen Receptor" or alternatively a "CAR" refers to a recombinant polypeptide construct comprising at least an extracellular antigen binding domain, a transmembrane domain and a cytoplasmic signaling domain (also referred to herein as "an intracellular signaling domain") comprising a functional signaling domain derived from a stimulatory molecule as defined below. In some embodiments, the domains in the CAR polypeptide construct are in the same polypeptide chain, e.g., comprise a chimeric fusion protein. In some embodiments, the domains in the CAR polypeptide construct are not contiguous with each other, e.g., are in different polypeptide chains. According to some embodiments, the CAR may broadly refer to any moiety that is expressed by the immune cell and has a cytotoxic effect on the target cancer cell, i.e. a ligand that activates a death receptor on the target.

The terms, "tumor environment", "tumor microenvironment" and "TME" may be used interchangeably and refer to the cellular environment in which the tumor exists, including surrounding blood vessels, immune cells, fibroblasts, bone marrow-derived inflammatory cells, lymphocytes, signaling molecules and the extracellular matrix (ECM).

The term "antigen" refers to a molecule that provokes an immune response. This immune response may involve antibody production, or the activation of specific immunologically-competent cells, or both. The skilled artisan will understand that any macromolecule, including virtually all proteins or peptides, can serve as an antigen. Furthermore, one skilled in the art will understand that an antigen need not be encoded solely by a full-length nucleotide sequence of a gene. It is readily apparent that the present invention includes, but is not limited to, the use of partial nucleotide sequences of more than one gene and that these nucleotide sequences are arranged in various combinations to encode polypeptides that elicit the desired immune response. It is readily apparent that an antigen can be generated synthesized or can be derived from a biological sample, or might be a macromolecule besides a polypeptide. Such a biological sample can include, but is not limited to, a tissue sample, a tumor sample, a cell or a fluid with other biological components.

The term "anti-tumor effect", refers to a biological effect which can be manifested by various means, including, but not limited to, e.g., a decrease in tumor volume, a decrease in the number of tumor cells, a decrease in the number of metastases, an increase in life expectancy, a decrease in tumor cell proliferation, a decrease in tumor cell survival, or amelioration of various physiological symptoms associated with the cancerous condition.

The term "autologous" refers to any material derived from the same individual to whom it is later to be re-introduced into the individual. The term "allogeneic" refers to any material derived from a different individual than to whom the material is introduced.

The term "conservative sequence modifications" refers to amino acid modifications that do not significantly affect or alter the binding characteristics of a factor thereto. Such conservative modifications include amino acid substitutions, additions and deletions.

As used herein, the term "Immune effector cell" refers to a cell that is involved in an immune response. Examples include various types, and sub-types of T cells, B cells, natural killer (NK) cells, Innate Lymphocyte Cells (ILCs), natural killer T (NKT) cells, Mast cells, Macrophage, Monocytes, Dendritic cells, Basophil, Neutrophils and Eosinophil.

The term "expression" refers to the transcription and/or translation of a particular nucleotide sequence driven by a promoter.

The term "expression vector" refers to a vector comprising a recombinant polynucleotide comprising expression control sequences operatively linked to a nucleotide sequence to be expressed. Expression vectors include all those known in the art, including cosmids, plasmids, episomes, transposons and viruses (e.g., lentiviruses, retroviruses, adenoviruses, and adeno-associated viruses) that incorporate the recombinant nucleotide sequences.

As used herein, the term "TME responsive expression vector" refers to an expression vector configured to express a gene product in the presence of factors constituting, defining or otherwise associated with a tumor environment.

As used herein, the term "promoter" refers to a DNA sequence recognized by the synthetic machinery of the cell, or introduced synthetic machinery, required to initiate the specific transcription of a polynucleotide sequence.

As used herein, the term "promoter/regulatory sequence" and "promoter response element (PRE)" may be used interchangeably and refer to nucleic acid sequences required for expression of a gene product operably linked to the promoter/regulatory sequence. As used herein, the term "TME factor" refers to a factor present and active in a TME such as but not limited to cytokines, transcription factors etc. As used herein, the term "PRE linked to a TME-associated factor refers to PRE that is activated following the excreted effect of the TME-associated factor. E.g "hypoxia PRE" refers to PRE that is activated following hypoxia in the TME. " IFN-γ PRE" refers to PRE that is activated following the presence of IFN-γ in the TME. In some instances, this sequence may be the core promoter sequence and, in other instances, this sequence may also include an enhancer sequence and other regulatory elements, which are required for expression of the gene product. The promoter/regulatory sequence may, for example, be one which expresses the gene product in a tissue specific manner.

As used herein, the term "constitutive" promoter refers to a nucleotide sequence which, when operably linked with a polynucleotide encoding a gene product, causes the gene product to be produced in a cell under most or all physiological conditions of the cell.

As used herein, the term "inducible" promoter refers to a nucleotide sequence which, when operably linked with a polynucleotide encoding a gene product, causes the gene product to be substantially enhanced only when an inducer is present. "Induction" may include both the initiation of expression from an OFF state into an ON state, as well as the enhancement of expression from relative-LOW to relative-HIGH.

As used herein, the terms "TME specific promoter", "TME inducible promoter" and "TME responsive promoter" may be used interchangeably and refer to a nucleotide sequence which causes the gene product to be induced within TME.

As used herein, the term "Synthetic promoter" refers to DNA sequences artificially synthesized as opposed to cloning of naturally occurring promoters.

The terms "cancer associated antigen" and "tumor antigen" may be used interchangeably and refer to a molecule (typically a protein, carbohydrate or lipid) that is expressed on the surface of a cancer cell, either entirely or as a fragment and which is useful for the preferential targeting of a pharmacological agent to the cancer cell. In some embodiments, a tumor antigen is a marker expressed by both normal cells and cancer cells. In some embodiments, a tumor antigen is a cell surface molecule that is overexpressed in a cancer cell in comparison to a normal cell, for instance, 1-fold over expression, 2-fold overexpression, 3-fold overexpression or more in comparison to a normal cell. In some embodiments, a tumor antigen is a cell surface molecule that is inappropriately synthesized in the cancer cell, for instance, a molecule that contains deletions, additions or mutations in comparison to the molecule expressed on a normal cell.

As used herein, the term "treating" refers to the reduction or amelioration of the progression, severity and/or duration of a proliferative disorder, or the amelioration of one or more symptoms (preferably, one or more discernible symptoms) of a proliferative disorder resulting from the treatment. In other embodiments the term refers to the inhibition of the progression of a proliferative disorder. In other embodiments, the term refers to the reduction or stabilization of tumor size or cancerous cell count. The term "transfected" refers to a process by which an exogenous nucleic acid is transferred or introduced into a host cell. The cell includes the primary subject cell and its progeny.

The terms "specifically binds" and "binding" refer to a factor such as a transcription-factor, which recognizes and binds a cognate nucleic acid sequence.

As used herein, the terms "substantially" and "essentially" with regards to the absence of gene expression in a non-tumor environment, i.e. in healthy tissue, may include no or residual expression levels only. According to some embodiments, substantially no expression (such as in healthy tissue) may refer to expression levels at levels that are biologically/functionally ineffective against normal healthy tissues, while inducing effective levels within TME.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99% identity, includes something with 95%, 96%, 97%, 98% or 99% identity, and includes sub-ranges such as 96-99%, 96-98%, 96-97%, 97-99%, 97-98% and 98-99% identity. This applies regardless of the breadth of the range.

### Description

According to the invention, there is provided a tumor microenvironment (TME) responsive expression vector comprising a nucleic acid sequence encoding a chimeric antigen receptor (CAR) operably linked to a synthetic promoter, said promoter comprising two or more different TME dependent promoter response elements (PRE); wherein said at least two PRE's are an interferon-gamma-(IFN-γ) PRE and an NEκB PRE, and wherein the IFN-γ promoter response element comprises the nucleic acid sequence set forth in SEQ ID NO: 1, and wherein the NEκB PRE comprises the sequence set forth in SEQ ID NO: 2 ("K") ; wherein said TME responsive expression vector is so designed, that the presence of IFN-γ and TNFα in the TME, when binding to interferon-gamma-(IFN-γ) PRE and NEκB PRE, induces a higher expression level of CAR than when only IFN-γ or TNFα are pre-sent in the TME, and minimal no or low expression of CAR occurs in the absence of IFN-γ and TNFα, so that the CAR is upregulated and directing activities against the tumor while sparing normal tissue.

It is understood that a trade-off may be made between including fewer response elements, so that a broad spectrum of cancers can be targeted utilizing a same TME responsive expression construct and including a more comprehensive combination of response elements increasing the specificity of the expression construct to tumor tissue as opposed to normal tissue.

According to some embodiments, the CAR is a chimeric antigen T-cell receptor (CAR-T) or a chimeric antigen Natural Killer (NK) cell receptor (CAR-NK).

According to the invention, the promoter response element includes/encompasses interferon-gamma (IFN-γ, G)-response elements/binding sites, one or more Nuclear Factor kappa-B (NF-κB, K)-response elements/binding sites, and optionally one or more heat shock protein 70 (HSP-70) response elements/binding sites, one or more hypoxia response (H) elements/binding sites, one or more Interleukin 6 (IL-6, J) response elements/binding sites or any combination thereof. Each possibility is a separate embodiment.

According to the invention, the promoter response element comprises two or more promoter response elements/binding sites. The two or more promoter response elements/binding sites include an NF-xB-response elements/binding site and an IFN-γ-response elements/binding site. According to some embodiments, binding of TME factors to the two or more TME dependent promoter response elements induces a higher expression level of CAR than binding to a single TME dependent promoter response element. As a non-limiting example, binding of NF-κB to the NF-xB-response elements/binding site and TNF-α to the IFN-γ-response elements/binding site of the promoter may, according to some embodiments, induce a higher expression of the CAR than binding of NF-κB or TNF-α alone.

According to the invention, the promoter response element comprises a nucleic acid selected from the group consisting of GGGAATTTCC set forth in SEQ ID NO. 2 (abbreviated herein as K), As a non-limiting example, the promoter response element comprises both the nucleic acid sequence TTCCGGGAA set forth in SEQ ID NO. 1 and the nucleic acid sequence GGGAATTTCC set forth in SEQ ID NO. 2 (abbreviated G1K1). According to some embodiments, the nucleic acids may be coextensive. As a non-limiting example, the nucleic acid sequence GACCTTGAGTACGTGCGTCTCTGCACGTATG set forth in SEQ ID NO. 3 may be immediately followed by the nucleic acid sequence GCGCTTCCTGACAGTGACGCGAGCCG set forth in SEQ ID NO. 4 (abbreviated H1J1. According to some embodiments, the nucleic acids may be separated by a spacer sequence. As a non-limiting example, the nucleic acid sequence TTCCGGGAA set forth in SEQ ID NO. 1 and the nucleic acid sequence GGGAATTTCC set forth in SEQ ID NO. 2 may be spaced apart by a spacer element within the same synthetic promoter.

According to some embodiments, the TME responsive expression vector further includes a nucleic acid sequence encoding an externally inducible promoter and a nucleic acid sequence encoding a trans-activator, e.g. rtTA3. According to some embodiments, the synthetic promoter drives expression of the trans-activator and the inducible promoter drives expression of the CAR. According to some embodiments, only the combined presence of the external inducer and the TME factor results in CAR expression. According to some embodiments, the presence of the external inducer in the absence of TME factor, causes substantially no induction of CAR expression. According to some embodiments, the presence of the external inducer in the absence of TME factor, causes minimal induction of CAR expression. According to some embodiments, when the TME factor binds the promoter response element in the absence of the external inducer, essentially no CAR expression is induced. According to some embodiments, a minor level of CAR expression is also found in the un-induced state. Such minimal expression may serve to ensure that CAR-T memory is maintained.

According to some embodiments, the inducible promoter may be a Tet-Response-Element promoter, and the external inducer may be doxycycline and/or tetracycline. According to some embodiments, the Tet-Response-Element may be activated by the combined presence of the trans-activator and doxycycline and/or tetracycline. The tetracycline (Tet)-On system is an inducible gene expression system for mammalian cells, in which the reverse Tet transactivator (rtTA) fusion protein, which is composed of the doxycycline-binding Tet-repressor mutant protein and the C-terminal activator domain from the herpes simplex virus VP16 protein, is engineered to control gene expression by providing doxycycline (Dox). In the presence of Dox, rtTA activates a minimal promoter that is fused downstream of an array (e.g. seven) repeated Tet-operator sequences. Until recently, all Tet-On systems had required two separate vectors, one to introduce rtTA and another with the inducible promoter to control the gene of interest. However, a one-vector system has recently been developed, which has enabled transduction of a gene of interest into primary immune cells. By utilizing this one-vector system, it is possible to control target expression and functions using the Tet-On inducible system.

According to some embodiments, the CAR molecule encoded by the CAR sequence comprises an antigen binding domain, a transmembrane domain, and an intracellular domain, optionally comprising a costimulatory domain and/or a primary signaling domain. According to some embodiments, the antigen binding domain binds to a tumor antigen. Non-limiting examples of tumor antigens include: thyroid stimulating hormone receptor (TSHR); CD171 ; CS- 1 (CD2 subset 1 , CRACC, SLAMF7, CD319, and 19A24); C-type lectin-like molecule- 1 (CLL- 1); ganglioside GD3 (aNeu5Ac(2- 8)aNeu5Ac(2-3)bDGalp(] -4)bDGlcp(l-l )Cer); Tn antigen (Tn Ag); Fms-Like Tyrosine Kinase 3 (FLT3); CD38; CD44v6; B7H3 (CD276); KIT (CD117); Interleukin- 13 receptor subunit alpha-2 (IL- 13Ra2); Interleukin 11 receptor alpha (ILl lRa); prostate stem cell antigen (PSCA); Protease Serine 21 (PRSS21); vascular endothelial growth factor receptor 2 (VEGFR2); Lewis(Y) antigen; CD24; Plateletderived growth factor receptor beta (PDGFR-beta); stage-specific embryonic antigen-4 (SSEA-4); Mucin 1, cell surface associated (MUC1); epidermal growth factor receptor (EGFR); neural cell adhesion molecule (NCAM); carbonic anhydrase IX (CAIX); Proteasome (Prosome, Macropain) Subunit, Beta Type, 9 (LMP2); ephrin type-A receptor 2 (EphA2); Fucosyl GM1 ; sialyl Lewis adhesion molecule (sLe); ganglioside GM3 (aNeu5Ac(2-3)bDGalp(l -4)bDGlcp(l -l)Cer; TGS5 ; high molecular weight- melanoma-associated antigen (HMWMAA); o-acetyl- GD2 ganglioside (OAcGD2); Folate receptor beta; tumor endothelial marker 1 (TEM1/CD248); tumor endothelial marker 7-related (TEM7R); claudin 6 (CLDN6); G protein-coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR- 1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen- 1 (MAD-CT-1); melanoma cancer testis antigen-2 (MAD- CT-2); Fos-related antigen 1 ; p53 mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N- Acetyl glucosaminyl-transferase V (NA17); paired box protein Pax-3 (PAX3); Androgen receptor; Cyclin B 1 ; v-myc avian myelocytomatosis viral oncogene neuroblastoma derived homolog (MYCN); Ras Homolog Family Member C (RhoC); Cytochrome P450 1B 1 (CYP1B 1); CCCTC-Binding Factor (Zinc Finger Protein)-Like (BORIS); Squamous Cell Carcinoma Antigen Recognized By T Cells 3 (SART3); Paired box protein Pax-5 (PAX5); proacrosin binding protein sp32 (OY-TES 1); lymphocyte-specific protein tyrosine kinase (LCK); A kinase anchor protein 4 (AKAP-4); synovial sarcoma, X breakpoint 2 (SSX2); CD79a; CD79b; CD72; Leukocyte- associated immunoglobulin-like receptor 1 (LAIR1); Fc fragment of IgA receptor (FCAR); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module-containing mucin-like hormone receptor- like 2 (EMR2); lymphocyte antigen 75 (LY75); Glypican-3 (GPC3); Fc receptor-like 5 (FCRL5); and immunoglobulin lambda- like polypeptide 1 (IGLL1) and any combination thereof. Each possibility is a separate embodiment.

According to some embodiments, the TME responsive expression vector further comprises effector-genes encoding a protein (or functional RNA) that enhance penetration of the immune effector cell in to the tumor, such as, but not limited to, proteases of the MMP8/9.

According to some embodiments, the TME responsive expression vector further comprises one or more effector-genes encoding miRNAs that suppress immuneinhibitors, such as, but not limited to, PD1 and/or CTLA4, within the tumor.

According to some embodiments, the TME responsive expression vector further comprises one or more effector-genes encoding cytokines bringing about immune-cell retention within the tumor, such as, but not limited to, CXCL9/10 and/or CRCR3 ligands, thereby generating an autocrine loop.

According to some embodiments, the vector may be any suitable vector allowing expression in mammalian cells, such as human cells. According to some embodiments, the vector may be selected from a DNA vector, an RNA vector, a plasmid, a lentivirus vector, an adenoviral vector, or a retrovirus vector. Each possibility is a separate embodiment.

According to some embodiments, a TME vector library may be created, which library includes TME vectors, each having a unique TME responsive expression element profile.

According to some embodiments, there is provided an immune effector cell or cell population comprising the hereindisclosed tumor environment (TME) responsive expression vector. According to some embodiments, the immune effector cell or cell population is an NK cell or a T-cell.

Disclosed herein is a method for treating cancer in a patient in need thereof, the method comprising administering an effective amount of an immune effector cell comprising the hereindisclosed tumor environment (TME) responsive expression vector.

The method further comprises administrating to the patient an external inducer, such as, but not limited to, tetracycline and/or doxycycline. The external inducer may be provided before, concurrently with, or after the administration of the immune effector cell having the hereindisclosed tumor environment (TME) responsive expression vector.

The method may include a step of evaluating CAR expression levels and/or checking the patient for adverse effects. The CAR expression levels may be evaluated before administering the external inducer. The CAR expression levels may be evaluated, during and/or after administrating the external inducer. As a non-limiting example, a first bolus of external inducer may be initially given, followed by an evaluation of CAR expression. A second bolus may then be administered based on the CAR expression level detected and the patient's response to the treatment. The external inducer may be provided repeatedly, for example every 10 hours, every day, every two days or any other suitable time interval. The administering of the external inducer may be terminated if adverse effects are detected. The amount of external inducer administered may be increased/decreased based on the evaluated CAR expression levels and/or based on the patient's response to the treatment.

Disclosed herein is a method for screening a patient for determination of an optimal synthetic promoter for CAR expression. The method includes obtaining a biopsy of a patient's tumor, determining the expression profile of one or more TME factors in the biopsy; and selecting and/or engineering a TME responsive expression vector having a TME dependent promoter response element matching the expression profile of the one or more TME factors in the biopsy. The TME may be expressed by the tumor cells and/or by non-tumor TME cells. The non-tumor TME cells may be the immune effector cells.

A tissue sample obtained from the patient's tumor and optionally also from healthy tissue may be grown *in-vitro* and a library of TME-responsive vectors and may be used to screen for the vector proving most effective and selective for treatment, namely a vector having a TME response profile matching that of the tumor. This to obtain maximum expression in tumor tissue, while also being unique to the tumor, so that no or minimal expression is obtained in healthy tissue.

The method disclosed herein further includes introducing the selected TME responsive expression vector into an immune effector cell or cell population. The immune effector cell or cell population is an NK cell or a T-cell. The immune effector cell or cell population is autologous to the patient. The immune effector cell or cell population are isolated from the patient prior to the treatment.

The method disclosed herein further includes administering the immune effector cell or cell population to the patient.

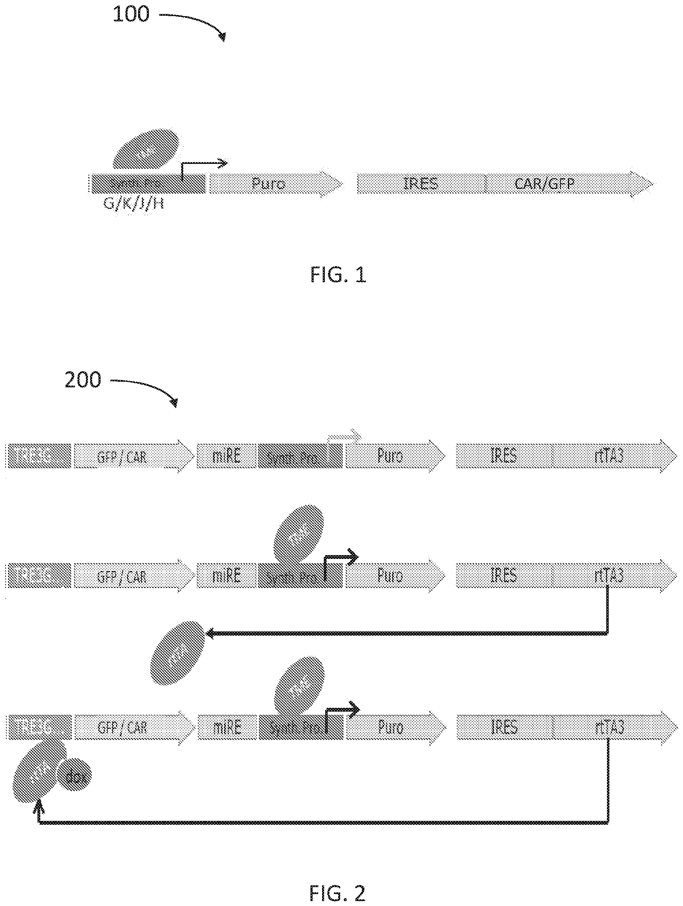

Reference is now made to **FIG. 1** which schematically illustrates an expression construct **100** comprising a synthetic promoter directly controlling expression of a CAR (or a reporter gene), according to some embodiments. Expression construct **100**, when introduced into a host immune effector cell, is configured to directly induce transcription of the CAR (or the GFP marker) in a tissue environment in which a TME factor, matching the TME response element of expression construct **100**, is prevalent.

Expression construct **100** includes the following elements:
**Synth.Pro.:** a synthetic promoter composed of (i) a minimal transcription promoter having a TATAA box that can initiate expression with adequate proximal elements; and (ii) unique combinations of promoter response elements, here IFN-γ response element (abbreviated "G"), NF-kB-response elements (abbreviated "K"), Hypoxia response Elements (abbreviated as "H"), and/or IL-6 response elements (abbreviated "J").

**TME:** marks the area of the promoter in which the promoter response element sequences are located and to which inducible factors present in the TME can bind, thereby activating the synthetic promoter (e.g. IFN-γ, TNF-α, Hypoxia and IL-6).

**Puro:** a resistance gene that is also transcribed by the synthetic promoter. The resistance gene may as here be shown to be a puromycin resistance gene; however other resistance genes are also applicable and within the scope of this disclosure. The resistance gene is introduced to enable positive *in vitro* selection of cells transduced with the vector.

**IRES:** Internal Ribosome Entry Site enables translation of both the resistance gene and the target gene (e.g. CAR or a GFP marker) from the same mRNA.

Reference is now made to **FIG. 2** which schematically illustrates an expression construct **200** comprising a synthetic promoter indirectly controlling expression of a CAR (or a reporter gene), according to some embodiments. Expression construct **200**, when introduced into a host immune effector cell, is configured to induce transcription of a transactivator in a tissue environment in which a TME factor, matching the TME response element of expression construct **200**, is prevalent. The transactivator will then induce expression of the CAR if an exogenously administered inducer (here doxycycline) is provided. Such indirect induction of CAR expression provides an ON-OFF safety mechanism, enabling cessation of CAR expression in case non-specific expression is detected or adverse effects observed.

Expression construct **200** includes the following elements:
**Synth.Pro.:** a synthetic promoter composed of (i) a minimal transcription promoter having a TATAA box that can initiate expression with adequate proximal elements; and (ii) unique combinations of promoter response elements, here IFN-γ response element (abbreviated "G"), NF-kB-response elements (abbreviated "K"), Hypoxia response Elements (abbreviated as "H"), and/or IL-6 response elements (abbreviated "J").

**TME:** marks the area of the promoter in which the promoter response element sequences are located and to which inducible factors present in the TME can bind, thereby activating the synthetic promoter (e.g. IFN-γ, TNF-α, Hypoxia and IL-6).

**Puro:** a resistance gene that is also transcribed by the synthetic promoter. The resistance gene may as here be shown to be a puromycin resistance gene; however other resistance genes are also applicable and within the scope of this disclosure. The resistance gene is introduced to enable positive *in vitro* selection of cells transduced with the vector.
**rtTA3:** trans-activator gene the transcription of which transcription is mediated by the synthetic promoter.

**IRES:** the IRES element enables transcription of both the resistance gene and the transactivator gene (here rtTA3) from the same synthetic promoter.

**Dox:** doxycycline, a potent analog of tetracycline, which serves as a co-activator to the trans-activator.

**TRE3G:** a Tet-Response-Element promoter that is activated by the combined presence of the rtTA3 and exogenous doxycycline and which mediates the transcription of CAR (or a reporter gene or other target gene). This is the basis of the enhancementsafety vector that, on the one hand, allows an amplified, TME specific CAR expression in conjunction with an additional safety layer by constructing the abovementioned synthetic promoter in front of the rtTA3 trans-activator and the reporter gene/CAR under the Tet-Response-Element promoter. The inclusion of rtTA-tet response provides an amplification of the transcription as it recruits the VP 16 transcription activator. In addition, the tetracycline/doxycycline-inducible element adds another layer of safety as it is active only in the presence of the tetracycline (or Dox), thus allowing it to turn the system OFF easily.
**miRE:** the micro-RNA element (miRE) is providing both transcription-ending for the TRE3G promoter-transcribed ORF and concurrent expression of miR for the silencing of other genes of interest (e.g. silencing of the endogenous T-cell Receptor or immune checkpoint receptors like PD-1).

Disclosed herein is a method for screening a promoter-library for having an optimal Tumor Micro-Environment (TME) expression pattern, i.e. promoters inducing strong expression in a TME, yet having little, if any, expression in non-TME.

The promoter library includes expression constructs with candidate synthetic promoters that are constructed and optionally tested functionally *in vitro.*

The promoters in the library have nucleic-acid sequences with expected binding-sites of transcription-factors that are activated within TMEs.

The promoter includes more than one candidate nucleic-acid sequence, the candidate nucleic acid sequences being spaced apart by nucleotide sequences (also referred to herein as "spacers").

The promoter further includes a minimal-promoter sequence followed by a reporter gene, such as fluorescent-proteins.

The construct includes two fluorescent reporters: a first reporter having a cryptic off-frame ATG-start codon, and a second reporter positioned after an Internal-Ribosome-Entry-Site (IRES) enabling independent translation thereof. The first fluorescent reporter will be poorly translated, yielding a signal only when highly transcribed, while the second fluorescent reporter is independently translated, also when transcribed at low-levels.

The library of candidate promoters includes promoters with a constant "core" portion, and variable nucleotides that are based on the binding-sites of the transcription factors of interest. Spacer-sequences, tandem-repeats of binding-sites, and the composition of various binding-sites of multiple factors are also enabling the generation of the libraries. The complexity may be limited by focusing variable-nucleotide on strategic positions, based on an analysis of multiple binding-sites of the transcription-factors of interest. The complexity may be increased by addition of variable nucleotides and/or repeats of binding-sites and/or the spacers between them.

As a non-limiting example, the core-sites with specific variable nucleotides may include, but not be limited to: AYTTCCSGGAART for STAT1-binding, and GGRRRTTYYC for NF-kB, where A=adenine, T=thymidine, C= cytidine, G=guanine, Y=C/T, S=C/G, R=A/G. Non-limiting examples of suitable spacers include: AGGGTGGGCAAGT, tctaga, GGGGACTTTCC.

The promoters are cloned into an expression-vector, such as, but not limited to, a pHage2 lentiviral vector with the above described first and second fluorescentreporters, as well as additional sequences needed for plasmid propagation and for the generation of lentiviral particles.

The synthetic promoter sequences may be cloned into the expression vector using any cloning technique. Non-limiting examples of suitable cloning techniques include "classical" cloning using restriction-enzymes, and in-fusion cloning.

Optionally, PCR-amplification of the promoter library using a non-proof-reader polymerase may be utilized to introduce random mutations, which may further increase the variability and complexity of the library.

Lentiviruses may be generated by common techniques, such as by transient co-transfection of the expression vector, together with packaging-plasmids, in 293T-HEK cells. Cell-lines of interest (e.g. 293 cells) may then be transduced with the virus (preferably at a MOI<0.3) to obtain a single copy per cell, and may afterwards be expanded as needed.

The method disclosed herein further includes identifying cells with optimal promoters, i.e. promoters inducing strong expression in a TME, yet having little if any expression in non-TME. The cells may be identified by growing the transduced cells with and without stimulus and in the presence/absence of the relevant TME factors, such as, but not limited to: TNF-alpha, IFN-gamma, Hypoxia, IL-6 and TGF-beta. The cells are then sorted (e.g. using FACS) according to their expression of the reporter genes. Cells with very-high expression that gain dual-colors of both the first and the second fluorescent reporters may be separated from cells with moderate-high expression of the second reporter only.

It is understood that other techniques may be used. For example, the first and second reporters may be antibiotic resistance genes in which case the sorting may be made identifying antibiotic resistant cells.

The sorted cells are further expanded without stimulating cytokines and then sorted for negative/low expression of the first reporter in order to avoid constitutiveactive promoters. Cells that show negative/low first reporter expression in the absence of stimulation are then further expanded.

The method disclosed herein may further include additional stimulation-sorting steps, by essentially repeating the positive and negative selections described above.

Once a desired-phenotype is obtained, the method disclosed herein may further include extracting genomic DNA from the cells (optionally after an additional expanding of the cells) using conventional molecular biology. The promoters of the vectors are extracted using PCR; for example, using primers specific to sequences upstream and downstream of the integrated library. The primers include additional extended portions allowing direct sequencing using commercial services.

The method disclosed herein further includes comparing the sequences of the enriched promoters to the sequences of the library, and optionally also to known promoters to identify novel optimal promoter sequences.

The method further includes validating the activity of the identified optimal promoters in the screened cell-line and/or in other cell-lines and/or in primary immunecells of interest.

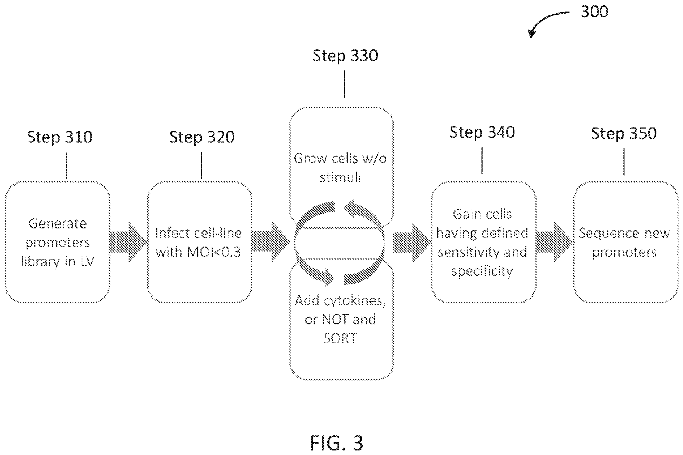

Reference is now made to **FIG. 3**, which is an illustrative flowchart **300** of the method for screening a promoters-library for providing an optimal Tumor Micro-Environment (TME) CAR-expression pattern.

In step **310** of the method a library (e.g. lentiviral (LV) library) including candidate promoters is constructed, as essentially described herein.

In step **320** cell lines (e.g. 293 cells) are infected with the lentiviruses of the library. Once cell lines expressing the promoter constructs are generated, in step **330**, the cells are grown in the presence or absence of stimuli (rtTA3 and doxycycline) and in the presence or absence of stimulating cytokines and then sorted (e.g. by FACS) according to their reporter gene expression profile, by separating cells with very-high expression that gain dual-colors of both the first and the second fluorescent reporters from cells with moderate-high expression of the TME dependent reporter only. As explained herein, this step may be repeated to further enhance the sensitivity and/or specificity of the promoter.

In step **340** cells having a desired expression profile (high expression of both promoters in the presence of stimuli and cytokines, while having little or no expression of the second), TME dependent reporter, in the absence of cytokines, are identified, and their promoter sequenced (step **350**).

The following examples illustrate the invention but should not be construed as limiting its scope, which is defined by the claims.

### EXAMPLES

### Example 1 - Defining response elements

Promoters, including the following response elements sequences, are listed in Table 1 below. The response elements were constructed just before a minimal transcription promoter with a TATAA box that can initiate expression with adequate proximal elements.

**Table 1. Promoter response element sequences**

| **Abbre viation** | **Response elements** | **Sequence** | **SEQ ID NO.** |
|---|---|---|---|
| K1 | 1xNF-κB | GGGAATTTCCGGGGACTTTCCGGGAATT TCCGGGGACTTTCCGGGAATTTCCAGAG CATATTAAGGTGACGCGTGTGGCCTCGA ACACCGAGCGACCCTGCAGCGACCCGC TTAAAAGCGGCCGCC | SEQ ID NO. 5 |
| G2 | 2xIFN-γ | TTCCGGGAAAGGGTGGGCAAGTTTCCG GGAAAGCAGTAGGTACAGCCTTCCGGG AAAGGGTGGGCAAGTTTCCGGGAAAGC AGTAGGTTTTTCGCATATTAAGGTGACG CGTGTGGCCTCGAACACCGAGCGACCCT GCAGCGACCCGCTTAAAAGGCGCGCC | SEQ ID NO. 6 |
| G4 | 4xIFN-γ | TTCCGGGAAAGGGTGGGCAAGTTTCCG GGAAAGCAGTAGGTACAGCCTTCCGGG AAAGGGTGGGCAAGTTTCCGGGAAAGC AGTAGGTACAGCCTTCCGGGAAAGGGT GGGCAAGTTTCCGGGAAAGCAGTAGGT ACAGCCTTCCGGGAAAGGGTGGGCAAG TTTCCGGGAAAGCAGTAGGTTTTCGCA TATTAAGGTGACGCGTGTGGCCTCGAAC ACCGAGCGACCCTGCAGCGACCCGCTT AAAAGGCGCGCC | SEQ ID NO. 7 |
| G6 | 6xIFN-γ | TTCCGGGAAAGGGTGGGCAAGTTTCCG GGAAAGCAGTAGGTACAGCCTTCCGGG AAAGGGTGGGCAAGTTTCCGGGAAAGC AGTAGGTACAGCCTTCCGGGAAAGGGT GGGCAAGTTTCCGGGAAAGCAGTAGGT ACAGCCTTCCGGGAAAGGGTGGGCAAG TTTCCGGGAAAGCAGTAGGTACAGCCTT CCGGGAAAGGGTGGGCAAGTTTCCGGG AAAGCAGTAGGTACAGCCTTCCGGGAA AGGGTGGGCAAGTTTCCGGGAAAGCAG TAGGTTTTCGCATATTAAGGTGACGCG TGTGGCCTCGAACACCGAGCGACCCTGC AGCGACCCGCTTAAAAGGCGCGCC | SEQ ID NO. 8 |
| G1K1 | 1xIFN-γ + 1×NF-κB | TTCCGGGAAAGGGTGGGCAAGTTTCCG GGAACCCGGGAATTTCCGGGGACTTTCC GGGAATTTCCGGGGACTTTCCGGGAATT TCCAGAGCATATTAAGGTGACGCGTGTG GCCTCGAACACCGAGCGACCCTGCAGC GACCCGCTTAAAAGCGGCCGCC | SEQ ID NO. 9 |
| G1K0.6 | 1xIFN-γ + 60% NF-κB | TTCCGGGAAAGGGTGGGCAAGTTTCCG GGAACCCGGGAATTTCCGGGGACTTTCC GGGAATTTCCAGAGCATATTAAGGTGA CGCGTGTGGCCTCGAACACCGAGCGAC CCTGCAGCGACCCGCTTAAAAGCGGCC GCC | SEQ ID NO. 10 |
| G2K2 | 2xIFN-γ + 2xNF-κB | TTCCGGGAAAGGGTGGGCAAGTTTCCG GGAAAGCAGTAGGTACAGCCTTCCGGG AAAGGGTGGGCAAGTTTCCGGGAAAGA GCAGGGAATTTCCGGGGACTTTCCGGG AATTTCCGGGGACTTTCCGGGAATTTCC AGAGCAGGGAATTTCCGGGGACTTTCC GGGAATTTCCGGGGACTTTCCGGGAATT TCCAGAGCATATTAAGGTGACGCGTGTG | SEQ ID NO. 11 |
| | | GCCTCGAACACCGAGCGACCCTGCAGC GACCCGCTTAAAAGGCGCGCC | |
| G3K3 | 3xIFN-γ + 3xNF-κB | TTCCGGGAAAGGTGGGCAAGTTTCCG GGAAAGCAGTAGGTACAGCCTTCCGGG AAAGGGTGGGCAAGTTTCCGGGAAAGC AGTAGGTACAGCCTTCCGGGAAAGGGT GGGCAAGTTTCCGGGAAAGAGCAGGGA ATTTCCGGGGACTTTCCGGGAATTTCCG GGGACTTTCCGGGAATTTCCAGAGCAG GGAATTTCCGGGGACTTTCCGGGAATTT CCGGGGACTTTCCGGGAATTTCCAGAGC AGGGAATTTCCGGGGACTTTCCGGGAAT TTCCGGGGACTTTCCGGGAATTTCCAGA GCATATTAAGGTGACGCGTGTGGCCTCG AACACCGAGCGACCCTGCAGCGACCCG CTTAAAAGGCGCGCC | SEQ ID NO. 12 |
| G3H2K 3 | 3xIFN-γ + 2xhypoxia+ 2xNF-κB | TTCCGGGAAAGGGTGGGCAAGTTTCCG GGAAAGCAGTAGGTACAGCCTTCCGGG AAAGGGTGGGCAAGTTTCCGGGAAAGC AGTAGGTACAGCCTTCCGGGAAAGGGT GGGCAAGTTTCCGGGAAAGCAGTAGGT ACAGCCGACCTTGAGTACGTGCGTCTCT GCACGTATGAGAGCAGACCTTGAGTAC GTGCGTCTCTGCACGTATGAGAGCAGG GAATTTCCGGGGACTTTCCGGGAATTTC CGGGGACTTTCCGGGAATTTCCAGAGCA GGGAATTTCCGGGGACTTTCCGGGAATT TCCGGGGACTTTCCGGGAATTTCCAGAG CAGGGAATTTCCGGGGACTTTCCGGGA ATTTCCGGGGACTTTCCGGGAATTTCCA GAGCATATTAAGGTGACGCGTGTGGCCT CGAACACCGAGCGACCCTGCAGCGACC CGCTTAAAAGGCGCGCC | SEQ ID NO. 13 |
| G3K3H 2 | 3xIFN-γ + 3xNF-κB + 2xhypoxia | TTCCGGGAAAGGGTGGGCAAGTTTCCG GGAAAGCAGTAGGTACAGCCTTCCGGG AAAGGGTGGGCAAGTTTCCGGGAAAGC AGTAGGTACAGCCTTCCGGGAAAGGGT GGGCAAGTTTCCGGGAAAGAGCAGGGA ATTTCCGGGGACTTTCCGGGAATTTCCG GGGACTTTCCGGGAATTTCCAGAGCAG GGAATTTCCGGGGACTTTCCGGGAATTT CCGGGGACTTTCCGGGAATTTCCAGAGC AGGGAATTTCCGGGGACTTTCCGGGAAT TTCCGGGGACTTTCCGGGAATTTCCAGA GCAGACCTTGAGTACGTGCGTCTCTGCA CGTATGAGAGCAGACCTTGAGTACGTG CGTCTCTGCACGTATGAGAGCATATTAA GGTGACGCGTGTGGCCTCGAACACCGA GCGACCCTGCAGCGACCCGCTTAAAAG GCGCGCC | SEQ ID NO. 14 |
| G2H2K 2 | 2xIFN-γ + 2xhypoxia+ 2xNF-κB | TTCCGGGAAAGGTGGGCAAGTTTCCG GGAAAGCAGTAGGTACAGCCTTCCGGG AAAGGGTGGGCAAGTTTCCGGGAAAGC AGTAGGTACAGCCGACCTTGAGTACGT GCGTCTCTGCACGTATGAGAGCAGACCT TGAGTACGTGCGTCTCTGCACGTATGAG AGCAGGGAATTTCCGGGGACTTTCCGG GAATTTCCGGGGACTTTCCGGGAATTTC CAGAGCAGGGAATTTCCGGGGACTTTCC GGGAATTTCCGGGGACTTTCCGGGAATT TCCAGAGCATATTAAGGTGACGCGTGTG GCCTCGAACACCGAGCGACCCTGCAGC GACCCGCTTAAAAGGCGCGCC | SEQ ID NO. 15 |
| G2K2H 2 | 2xIFN-γ + 2xNF-κB + 2xhypoxia | TTCCGGGAAAGGGTGGGCAAGTTTCCG GGAAAGCAGTAGGTACAGCCTTCCGGG AAAGGGTGGGCAAGTTTCCGGGAAAGA | SEQ ID NO. 16 |
| | | GCAGGGAATTTCCGGGGACTTTCCGGG AATTTCCGGGGACTTTCCGGGAATTTCC AGAGCAGGGAATTTCCGGGGACTTTCC GGGAATTTCCGGGGACTTTCCGGGAATT TCCAGAGCAGACCTTGAGTACGTGCGTC TCTGCACGTATGAGAGCAGACCTTGAGT ACGTGCGTCTCTGCACGTATGAGAGCAT ATTAAGGTGACGCGTGTGGCCTCGAAC ACCGAGCGACCCTGCAGCGACCCGCTT AAAAGGCGCGCC | |
| H2G2K 2 | 2xhypoxia + 2xIFN-γ + 2xNF-κB | GACCTTGAGTACGTGCGTCTCTGCACGT ATGAGAGCAGACCTTGAGTACGTGCGT CTCTGCACGTATGAGAGCATTCCGGAA AGGGTGGGCAAGTTTCCGGGAAAGCAG TAGGTACAGCCTTCCGGGAAAGGGTGG GCAAGTTTCCGGGAAAGAGCAGGGAAT TTCCGGGGACTTTCCGGGAATTTCCGGG GACTTTCCGGGAATTTCCAGAGCAGGG AATTTCCGGGGACTTTCCGGGAATTTCC GGGGACTTTCCGGGAATTTCCAGAGCAT ATTAAGGTGACGCGTGTGGCCTCGAAC ACCGAGCGACCCTGCAGCGACCCGCTT AAAAGGCGCGCC | SEQ ID NO. 17 |
| G1H2K 1 | 1xIFN-γ + 2xhypoxia + 1xNF-κB | TTCCGGGAAAGGGTGGGCAAGTTTCCG GGAAAGCAGTAGGTACAGCCGACCTTG AGTACGTGCGTCTCTGCACGTATGAGAG CAGACCTTGAGTACGTGCGTCTCTGCAC GTATGAGAGCAGGGAATTTCCGGGGAC TTTCCGGGAATTTCCGGGGACTTTCCGG GAATTTCCAGAGCATATTAAGGTGACGC GTGTGGCCTCGAACACCGAGCGACCCT GCAGCGACCCGCTTAAAAGGCGCGCC | SEQ ID NO. 18 |
| G1J1H 1 | 1xIFN-γ + 1xIL-6 + 1xhypoxia | TTCCGGGAAAGGGTGGGCAAGTTTCCG GGAACCCGACCTTGAGTACGTGCGTCTC TGCACGTATGTACAGCGCTTCCTGACAG TGACGCGAGCCGAGAGCATATTAAGGT GACGCGTGTGGCCTCGAACACCGAGCG ACCCTGCAGCGACCCGCTTAAAAGCGG CCGCC | SEQ ID NO. 19 |
| G1K0.6 J1 | 1xIFN-γ + 60% NF-κB + 1xIL-6 | TTCCGGGAAAGGGTGGGCAAGTTTCCG GGAACCCGGGAATTTCCGGGGACTTTCC GGGAATTTCCTACAGCGCTTCCTGACAG TGACGCGAGCCGAGAGCATATTAAGGT GACGCGTGTGGCCTCGAACACCGAGCG ACCCTGCAGCGACCCGCTTAAAAGCGG CCGCC | SEQ ID NO. 20 |
| G1K0.6 H1 | 1xIFN-γ + 60% NF-κB + 1xhypoxia | TTCCGGGAAAGGGTGGGCAAGTTTCCG GGAACCCGGGAATTTCCGGGGACTTTCC GGGAATTTCCTACAGACCTTGAGTACGT GCGTCTCTGCACGTATGAGAGCATATTA AGGTGACGCGTGTGGCCTCGAACACCG AGCGACCCTGCAGCGACCCGCTTAAAA GCGGCCGCC | SEQ ID NO. 21 |

The factors or combination of factors binding to the response elements listed in Table 1 (e.g. TNF-α, NF-κB and IL-6) are characteristic for many TME, and certain combinations of these factors may specifically represent a TME signature/profile.

However, it is noted, that the aforementioned factors may not be present in all TMEs. Similarly, additional factors may also be found in certain TMEs. Accordingly, other response elements may be included or may substitute the aforementioned response elements, and such additions and/or substitutions are within the scope of this disclosure.

Moreover, as explained herein, it is understood that a trade-off may be made between including less or more response elements. For example, including fewer types of response elements may enable targeting the expression to a broad spectrum of cancers, utilizing a same TME responsive expression construct. As another example, including a more comprehensive/exhaustive combination of response elements may increase the specificity of the expression construct to tumor tissue as opposed to normal/healthy tissue.

### Example 2 - Controlling promoter leakiness:

A major aspect of the invention is gaining an endogenous-induction of immuneeffector genes within TME. The synthetic promoter disclosed herein is configured to induce the expression within TME, while substantially limiting expression in healthy tissues. Therefore, the leakiness of the synthetic promoters was evaluated.

Two types of leakiness were envisaged.

The first type of leakiness manifests as transcription from the TME stimulated promoter, in the absence of TME factors, whether of CAR/GFP reporter, as in the case of expression construct **100** of **FIG. 1** or of the transactivator, as in the case of expression construct **200** of **FIG. 2**. This type of leakiness may be due to induction of the minimal promoter, without an exogenously provided TME-related stimulus, either because the cells tested endogenously express the factors, or due to promoter leakiness *per se.*

The second type of leakiness level, relevant for the expression constructs including a Tet-Response-Element promoter, such as expression construct **200** of **FIG. 2**, refers to expression of CAR/GFP reporter in the absence of doxycycline/tetracycline. This type of leakiness may be due to (i) presence of residual tet/dox in media or sera used and thus be an artifact of the *in-vitro* setting; or (ii) promoter leakiness *per se.*

HEK293T cells were transduced with the expression vectors disclosed in FIG. 2 and the following response elements:
1. G2 (2×IFN-γ),
2. G1K0.6 (combination of 1×IFN-γ and 60% of NF-κB sequence element), or
3. G3K3 (combination of 3xIFN-y and 3×NF-κB).

The cells underwent selection with puromycin to ensure the survival of only vector-transduced cells.

The GFP-intensity as well as the percentage of GFP-positive cells were tested.

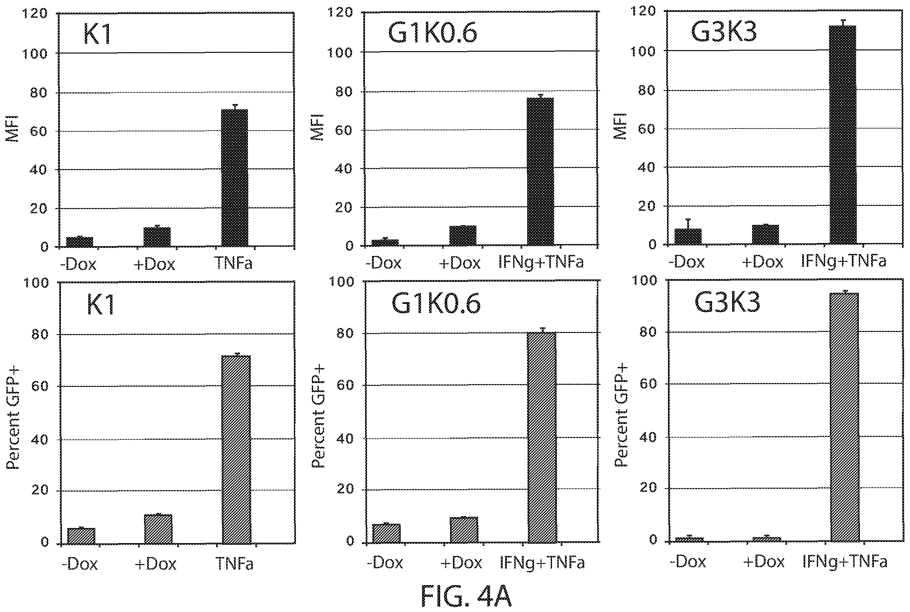

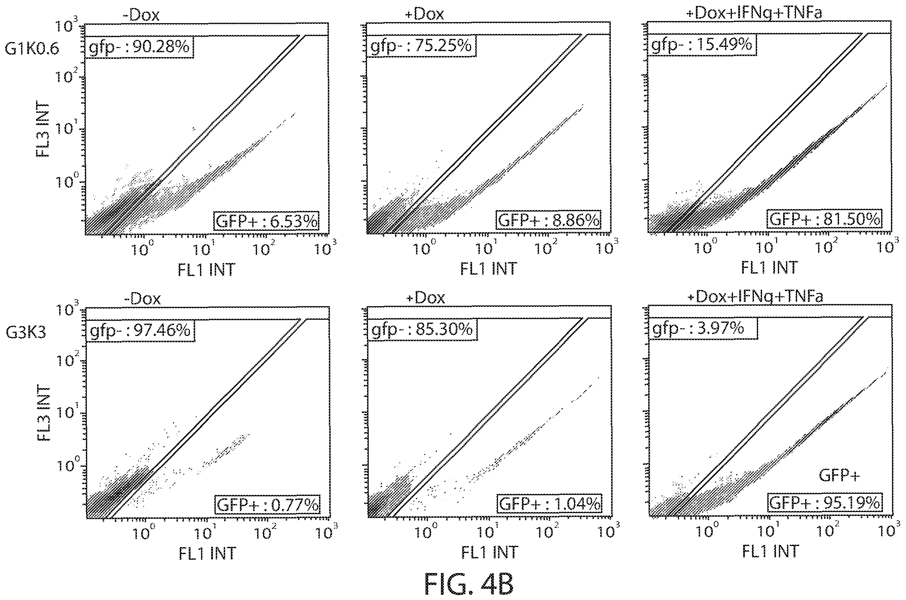

As seen from the histograms of **FIG. 4A** as well as in the representative FACS plot in **FIG. 4B**, in all three instances, adding doxycycline to the cell media did not, by itself, induce GFP expression (i.e. no increase in GFP intensity or in the percentage of GFP-positive cells was observed). However, in the combined presence of Doxycycline and TNF-α and/or IFN-γ, GFP intensity as well as the percentage of GFP-positive cells were markedly increased.

These results clearly demonstrate that the herein disclosed expression constructs enable TME specific expression.

### Example 3 - Response element synergism

It was hypothesized that including combinations of TME responsive elements would provide a synergistic expression.

The effect of including a number of IFN-γ elements (2, 4, and 6) as compared to a single IFN-γ element, reached a plateau already after two IFN-γ elements, both in the frequency of GFP-positive cells and in the GFP intensity (data not shown).

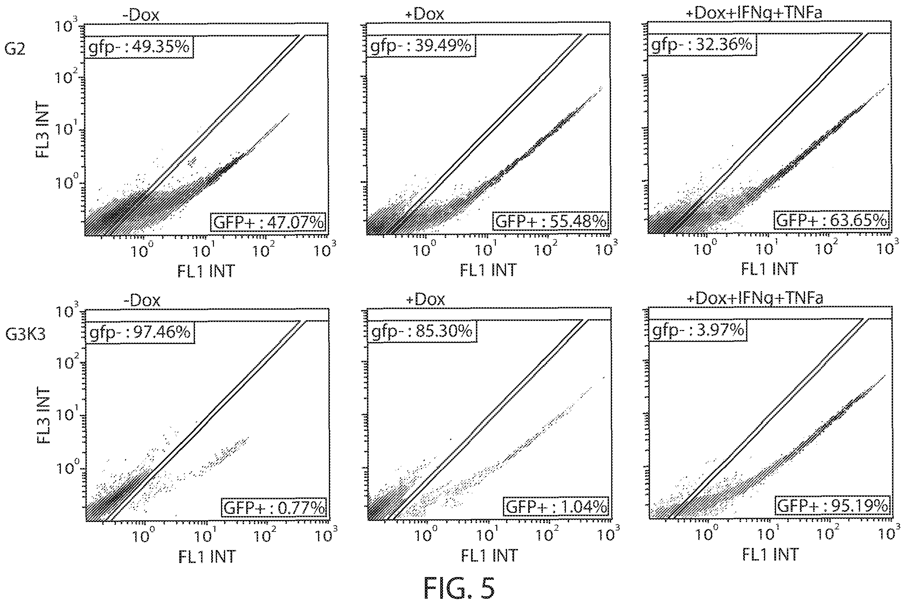

However, as seen from **FIG. 5**, a synergism was advantageously observed when NF-κB responsive elements were added to the IFN-γ elements, in that a promoter including three IFN-γ element response elements and three NF-κB response elements provided significantly higher percentages of GFP positive cells than a promoter including only IFN-γ response elements.

### Example 4 - TME profiling

Intensity and frequency of GFP expression, upon stimulus with a combination of inflammatory cytokines versus stimulus with single cytokines, was also tested. In short, HEK293T cells were transduced with vectors containing one of the following synthetic promoters:
1. G1K0.6: 1×IFN-γ and 60% of NF-κB,
2. G3K3: combination of 3×IFN-γ and 3×NF-κB.

This time the cells were harvested without prior puromycin selection, and the results thus represent the entire cell population, transduced as well as non-transduced cells.

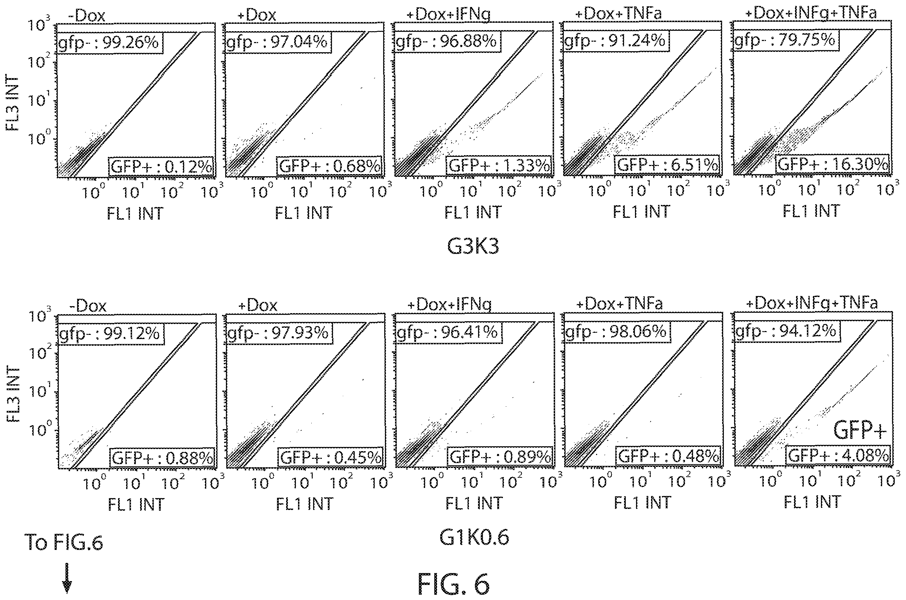

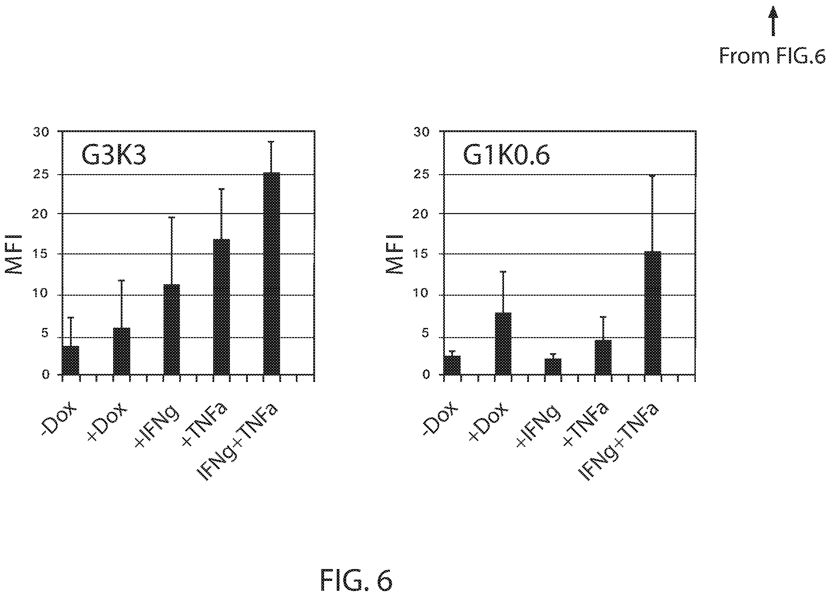

**FIG. 6** shows representative FACS plots (upper panels), gating GFP-positive cells (GFP+), as well as histograms (lower panels) showing a quantification of the GFP intensity (Median) for each tested condition.

Notably, transducing cells with the expression construct, having a G3K3 response element, showed cumulative expression, per cytokine added. In cells transduced with the expression construct having a G1K0.6 response element, adding each of IFN-γ and TNF-α separately caused only low levels of expression, whereas combined treatment with both IFN-γ and TNF-α induced substantial higher expression levels.

These results clearly show that controlled levels of expression can be achieved using the hereindisclosed expression constructs. Preferably, the construct utilized should be accustomed to the TME profile of the cells treated/targeted so as to optimize the expression level based on need.

### Example 5 - 3-factor response element

Following the study with combinations of two response elements IFN-γ and NF-κB (G and K), response elements including binding sites of additional factors, namely IL-6 and hypoxia (J and H), were also evaluated essentially as described above.

HEK293T cells were transduced with vectors containing one of the following synthetic promoters:
1. G1K0.6J1: 1×IFN-γ, 60% of NF-κB, 1×IL-6 response element sequences.
2. G1K0.6H1: 1×IFN-γ, 60% of NF-κB, 1×Hypoxia response element sequences.

GFP intensity and frequency of expression, was evaluated by FACS before and after stimulation with the indicated factors. Cells were harvested without selection.

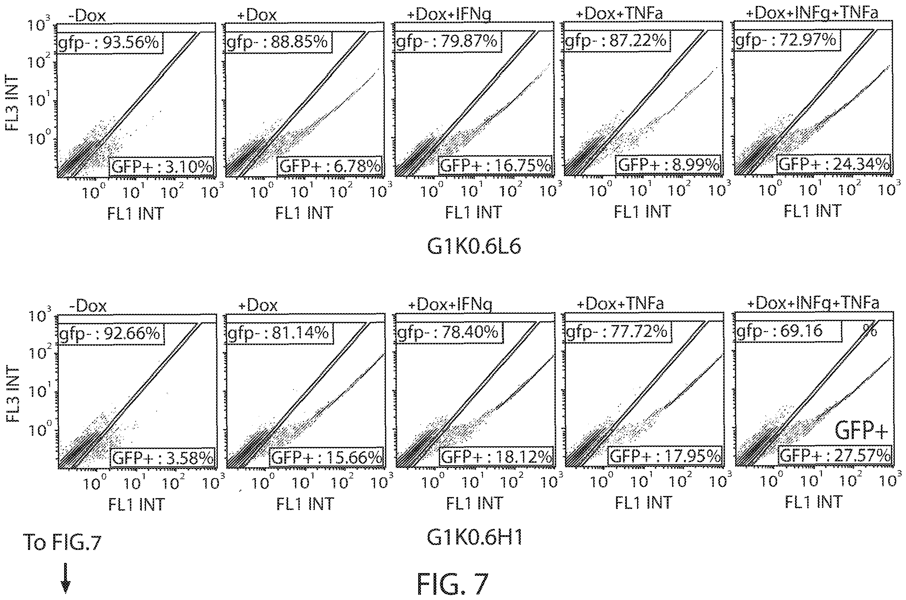

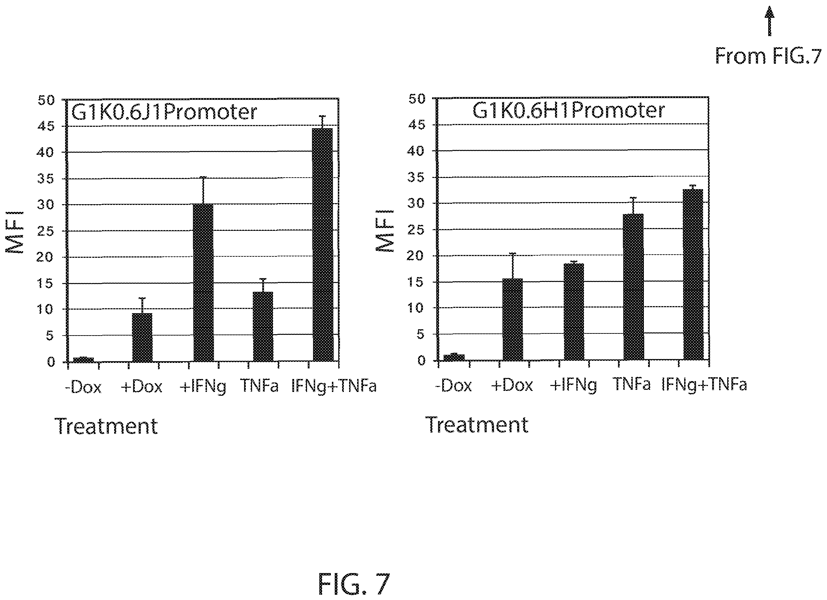

**FIG. 7** shows FACS plots (upper panels) of GFP-positive cells (GFP+) for each promoter and on each condition and histogram (lower panels) depicting quantification of the expression intensity (as Median) of the GFP-positive cells at each condition.

As seen from **FIG. 7**, the insertion of a third response element downstream to G1K0.6 elements did not further contribute to its synergistic effect. It is noted that this may not necessarily be a general observation but rather be representative of the specific cell line tested.

### Example 6 - screening for optimal promoter sequences

In order to identify promoters providing an optimal CAR expression profile, a lentiviral library of candidate promoters was constructed. The library includes constructs with promoters having a constant "core" portion, and variable nucleotides that are based on the binding-sites of the transcription factors of interest. Spacer-sequences, tandem-repeats of binding-sites, and the composition of various binding-sites of multiple factors are also included. The variability of the promoters was generated by changing, adding and/or deleting nucleotides at strategic and/or random positions of the promoters. Complexity was further increased by varying the number of binding-site repeats and/or the spacers between them. A total of 65,536 promoter sequences were included in the screen.

The constructs of the library include a fluorescent reporter (GFP) having a cryptic off-frame ATG-start codon ensuring that the reporter gene is only translated in the presence of high level of transcripts, i.e. high levels of transcription factor, here TME transcription factors. The constructs also include a second reporter (DsRed) positioned after an Internal-Ribosome-Entry-Site (IRES) enabling independent translation thereof, also at low levels of transcripts.

The constructs were then cloned into lentiviral vectors, and viruses were generated by transient co-transfection of the lentiviral vector together with packaging-plasmids into 293T-HEK cells.

293 cells were then infected (transduced) with the lentiviruses to obtain cell lines having incorporated into their genome a promoter construct of the library.

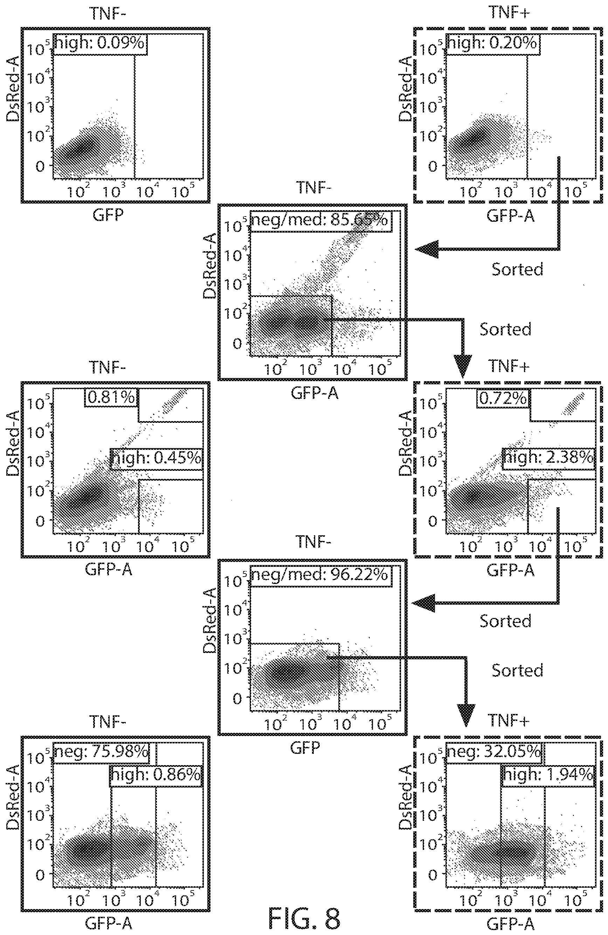

Subsequently, the transduced cells were then grown for 48h in the presence or absence of 250U/mL TNF, IFN or TNF and IFN and subsequently subjected to FACS sorting. Initially, cells showing high GFP expression or high GFP and DsRed expression in the presence of TNF, IFN or TNF and IFN were gated ("positive" sorting). As seen from the upper panel of **FIG. 8**, in the absence of cytokines, only 0.09% of the cells showed high GFP expression, while in the presence of TNF, IFN or TNF and IFN 0.2% of the cells had high GFP expression.

The gated cells were then subjected to a round of "negative" sorting after having been grown in the absence of cytokines (**FIG. 8** second panel). During this sorting step, cells having no or little GFP expression in the absence of cytokines were acquired, whereas cells with promoters causing constitutive expression were discarded (or separately sorted).

The positive and negative rounds of sorting were repeated twice (**FIG. 8** 3^{rd} and 4^{th} panels) until a final population, being 1.94% of the initial population, was acquired, which population being characterized by having high GFP expression, i.e. having an expression pattern highly sensitive to the presence/absence of TNF and/or IFN.

The acquired cells were propagated, and their promoter was sequenced using promoter specific primers. The screen identified 19 sequences out of the 65,536 sequences included in the screen. 18 were essentially similar but differed in 16 nucleotides (bold and underlined) plus in some instances some additional more random changes. An additional sequence (SEQ ID NO: 41) somewhat different from the other 18 was also retrieved

Table 2 below provides the sequences of promoters providing an optimal CAR expression profile, retrieved from the screen.

**Table 2 - promoter sequences**

| **SEQ ID NO.** | **Sequence** |
|---|---|
| 22 | TACAGGGACAGCAGAGATCCAGTTTGGACTAGCCCGGTCGCAC TAGTTCTAGAACTTCCCGGAAATAGGGTGGGCAAGTATTTCCG GGAAATTCTAGAGGGAGTTCCCGGGGACTTTCCGGGGATTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 23 | TACAGGGACAGCAGAGATCCAGTTTGGACTAGCCCGGTCGCAC TAGTTCTAGAACTTCCCGGAAGTAGGGTGGGCAAGTACTTCCC GGAAGTTCTAGAGGAAATTTTCGGGGACTTTCCGGGGGTTCTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 24 | TACAGGGACAGCAGAGATCCAGTTTGGACTAGCCCGGTCGCAC TAGTTCTAGAACTTCCCGGAAGTAGGGTGGGCAAGTACTTCCG GGAAATTCTAGAGGGAGTTCTCGGGGACTTTCCGGGAATTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 25 | TACAGGGACAGCAGAGATCCAGTTTGGACTAGCCGGTCGCAC TAGTTCTAGAACTTCCCGGAAGTAGGGTGGGCAAGTATTTCCC GGAAGTTCTAGAGGAAGTTCTCGGGGACTTTCCGGAGATTCTC TCTAGATATTAAGGTGACGCGTGTGGCCTCGAACACCGAGCGA CCCTGCAGCGACCCGCTTAAAAGCGGCCGCCATGGCCGCCAT GGCCTCCTCCGAGGACGTCATCAAGGAGTTCATGC |
| 26 | TACAGGGACAGCAGAGATCCAGTTTGGACTAGCCCGGTCGCAC TAGTTCTAGAACTTCCGGGAAATAGGGTGGGCAAGTACTTCCG GGAAATTCTAGAGGGGATTTCCGGGGACTTTCCGGAGGTTCTC TCTAGATATTAAGGTGACGCGTGTGGCCTCGAACACCGAGCGA |
| | CCCTGCAGCGACCCGCTTAAAAGCGGCCGCCATGGGCCGCCAT GGCCTCCTCCGAGGACGTCATCAAGGAGTTCATGC |
| 27 | TACAGGGACAGCAGAGATCCAGTTTGGACTAGCCCGGTCGCAC TAGTTCTAGAACTTCCGGGAAATAGGGTGGGCAAGTACTTCCG GGAAGTTCTAGAGGAGGTTTTCGGGGACTTTCCGGAGGTTTCC TCTAGATATTAAGGTGACGCGTGTGGCCTCGAACACCGAGCGA CCCTGCAGCGACCCGCTTAAAAGCGGCCGCCATGGCCGCCAT GGCCTCCTCCGAGGACGTCATCAAGGAGTTCATGC |
| 28 | TACAGGGACAGCAGAGATCCAGTTTGGACTAGCCCGGTCGCAC TAGTTCTAGAACTTCCGGGAAGTAGGGTGGGCAAGTATTTCCC GGAAGTTCTAGAGGGGGTTTTCGGGGACTTTCCGGAGGTTTCC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 29 | TACAGGGACAGCAGAGATCCAGTTTGGACTAGCCCGGTCGCAC TAGTTCTAGAACTTCCGGGAAGTAGGGTGGGCAAGTATTTCCG GGAAATTCTAGAGGGAGTTCTCGGGGACTTTCCGGGGATTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 30 | TACAGGGACAGCAGAGATCCAGTTTGGACTAGCCCGGTCGCAC TAGTTCTAGAATTTCCCGGAAATAGGTGGGCAAGTACTTCCG GGAAATTCTAGAGGGGGTTTCCGGGGACTTTCCGGGGGTTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 31 | TACAGGGACAGCAGAGATCCAGTTTGGACTAGCCCGGTCGCAC TAGTTCTAGAATTTCCCGGAAATAGGTGGGCAAGTATTTCCC GGAAATTCTAGAGGGGGTTTTCGGGGACTTTCCGGGAATTTTC TCTAGATATTAAGGTGACGCGTGTGGCCTCGAACACCGAGCGA CCCTGCAGCGACCCGCTTAAAAGCGGCCGCCATGGCCGCCAT GGCCTCCTCCGAGGACGTCATCAAGGAGTTCATGC |
| 32 | TACAGGGACAGCAGAGATCCAGTTTGGACTAGCCCGGTCGCAC TAGTTCTAGAATTTCCCGGAAATAGGTGGGCAAGTATTTCCG GGAAATTCTAGAGGAGGTTCTCGGGGACTTTCCGGGAGTTTTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 33 | TACAGGGACAGCAGAGATCCAGTTTGGACTAGCCCGGTCGCAC TAGTTCTAGAATTTCCCGGAAATAGGTGGGCAAGTATTTCCG GGAAATTCTAGAGGAGGTTTTCGGGGACTTTCCGGGAGTTTCC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 34 | TACAGGGACAGCAGAGATCCAGTTTGGACTAGCCCGGTCGCAC TAGTTCTAGAATTTCCCGGAAATAGGTGGGCAAGTATTTCCG GGAAGTTCTAGAGGAAGTTTTCGGGGACTTTCCGGGAATTTTC TCTAGATATTAAGGTGACGCGTGTGGCCTCGAACACCGAGCGA CCCTGCAGCGACCCGCTTAAAAGCGGCCGCCATGGCCGCCAT GGCCTCCTCCGAGGACGTCATCAAGGAGTTCATGC |
| 35 | TACAGGGACAGCAGAGATCCAGTTTGGACTAGCCCGGTCGCAC TAGTTCTAGAATTTCCGGGAAATAGGGTGGGCAAGTATTTCCC GGAAGTTCTAGAGGAGATTCTCGGGGACTTTCCGGGGGTTCTC TCTAGATATTAAGGTGACGCGTGTGGCCTCGAACACCGAGCGA CCCTGCAGCGACCCGCTTAAAAGCGGCCGCCATGGGCCGCCAT GGCCTCCTCCGAGGACGTCATCAAGGAGTTCATGC |
| 36 | TACAGGGACAGCAGAGATCCAGTTTGGACTAGCCCGGTCGCAC TAGTTCTAGAATTTCCGGGAAGTAGGGTGGGCAAGTACTTCCG GGAAATTCTAGAGGGGGTTTCCGGGGACTTTCCGGAGATTCTC TCTAGATATTAAGGTGACGCGTGTGGCCTCGAACACCGAGCGA CCCTGCAGCGACCCGCTTAAAAGCGGCCGCCATGGCCGCCAT GGCCTCCTCCGAGGACGTCATCAAGGAGTTCATGC |
| 37 | TACAGGGACAGCAGAGATCCAGTTTGGACTAGCCCGGTCGCAC TAGTTCTAGAATTTCCGGGAAATAGGGTGGGCAAGTATTTCCC GGAAGTTCTAGAGGGGGTTTTCGGGGACTTTCCGGAAATTTTC |
| | TCTAGATATTAAGGTGACGCGTGTGGCCTCGAACACCGAGCGA CCCTGCAGCGACCCGCTTAAAAGCGGCCGCCATGGGCCCCATG GCCTCCTCCGAGGACGTCATCAAGGAGTTCATGC |
| 38 | TACAGGGACAGCAGAGATCCAGTTTGGACTAGCCCGGTCGCAC TAGTTCTAGAACTTCCCGGAAATAGGTGGGCAAGTATTTCCC GGAAGTTCTAGAGGAGGTTTTCGGGGACTTTCCGGGATTCCCT CTAGATATTAAGGTGACGCGTGTGGCCTCGAACACCGAGCGAC CCTGCAGCGACCCGCTTAAAAGCGGCCGCCATGGCCGCCATG GCCTCCTCCGAGGACGTCATCAAGGAGTTCATGC |
| 39 | TACAGGGACAGCAGAGATCCAGTTTGGACTAGCCCGGTCGCAC TAGTTCTAGTTCCGGGAAGTGGGTGGGCAATATTTCCCGGAAG TTTAGAGGAAGTTTTCGGGGACTTCCGGAAATTCCCTCTAGAT ATTAAGGTGACGCGTGTGGCCTCGAACACCGAGCGACCCTGCA GCGACCCGCTTAAAAGCGGCCGCCATGGGCCGCCATGGCCTCC TCCGAGGACGTCATCAAGGAGTTCATGC |
| 40 | TACAGGGACAGCAGAGATCCAGTTTGGACTAGCCCGGTCGCAC TAGTTCTAGAACTTCTCGGAAATAGGGTGGGCAAGTACTGTGG CCTCGAACACCGAGCGACCCTGCAGCGACCCGCTTAAAAGCGG CCGCCATGGGCCGCCATGGCCTCCTCCGAGGACGTCATCAAGG AGTTCATGCAGACCAAGTCTCTGCTACC |

### Example 7 - Designing new PRE-based libraries and combining basic sequence or library-optimized sequence with other PREs into the same synthetic promoter

To generate a library of a specific PRE, either alone or combined with other PREs we took the following general approach, here described in detail while using the hypoxia PRE element as an example.

PRE sequences utilized in hypoxia dependent promoters of different genes were collected.

Based on the sequences a basic, non-naturally occurring hypoxia PRE element was generated by taking portions from natural occurring sequences suggested to function as hypoxia PRE in different genes. The portions include:
HBS sequence (ACGTG) found for example in the hypoxia dependent LDHA/EPO/VEGF genes.

Linker 1 (GCTGGAGT) an 8-nucleotide linker, not found in the promoters of natural target genes (not part of LDHA/EPO/VEGF genes).

HAS (CACAG) found for example in the hypoxia dependent EPO gene.

Linker 2 (TCCTCTT) a 7-nucleotide linker, not found in the promoters of natural target genes (not part of LDHA/EPO/VEGF genes).

These sequences were linearly combined into one sequence and then doubled in tandem to obtain the sequence set forth in SEQ ID. NO. 42

>ACGTGGCTGGAGTCACAGTCCTCTTACGTGGCTGGAGTCAC
AGTCCTCTT

Representing a "basic hypoxia PRE". It is understood that the basic hypoxia PRE is exemplary only and that other combinations, linkers, number of PREs as well as their orientations can also be envisaged.

The "basic hypoxia sequence" can then be added to additional PRE sequences (of the same or a different TME) to generate modified/alternative synthetic promoters. Non-limiting examples of optional modified/alternative synthetic promoters based on a basic TME factor (here hypoxia) PRE include
(i) Single or combined PREs,
   As a non-limiting example, the basic hypoxia PRE may be combined with G1K1 sequence (set forth in SEQ ID NO. 9), thereby generating the sequence set forth in SEQ ID NO. 46 (also referred to as H1G1K1). 
>ACGTGGCTGGAGTCACAGTCCTCTTACGTGGCTGGAGTCAC
AGTCCTCTTACTAGTTCTAGAACTTCCCGGAAGTAGGGTGGG
CAAGTACTTCCCGGAAGTTCTAGAGGAAATTTTCGGGGACTT
TCCGGGGGTTCTCTCTAGATATTAAGGTGACGCGTGTGGCCT
CGAACACCGAGCGACCCTGCAGCGACCCGCTTAAAAGCGGC
 
CGCCATGGGCCGCCATGGCCTCCTCCGAGGACGTCATCAAG
GAGTTC
(ii) Alternative positioning: the basic TME factor PRE can be added 5 prime and/or 3 prime and/or the middle of the synthetic promoter.
(ii) Alternative orientation: the basic TME factor PRE can be flipped and added as above in (i) and (ii).
   As a non-limiting example the basic hypoxia PRE may be combined with G1K1 sequence (set forth in SEQ ID NO. 9) in a flipped configuration thereby generating the sequence set forth in SEQ ID NO. 47 (also referred to as H1flipG1K1. 
>AAGAGGACTGTGACTCCAGCCACGTAAGAGGACTGTGACT
CCAGCCACGTACTAGTTCTAGAACTTCCCGGAAGTAGGGTG
GGCAAGTACTTCCCGGAAGTTCTAGAGGAAATTTTCGGGGA
CTTTCCGGGGGTTCTCTCTAGATATTAAGGTGACGCGTGTGG
CCTCGAACACCGAGCGACCCTGCAGCGACCCGCTTAAAAGC
GGCCGCCATGGGCCGCCATGGCCTCCTCCGAGGACGTCATC
AAGGAGTTC
(iv) Alternative Sequence. Nucleotides of the basic TME factor PRE sequence can be substituted. For example, as set forth in SEQ ID NO. 43.

>RCGTGSCTGGAGTMACAGTCCTCTTRCGTGSCTGGAGTMAC
AGTCCTCTT

Following the generation of the library, the library screen for the best sequences; i.e. the sequences that induce the less leakiness and the highest response to hypoxia stimulation, as essentially described in Example 6.

It is understood that the described approach for designing "basic response elements" and then generating a library of modified/alternative PREs based on the "basic response element" - may be adapted for all of the herein described TME factor PREs.

## Claims

1. A Tumor Micro-Environment (TME) responsive expression vector comprising:
a nucleic acid sequence encoding a chimeric antigen receptor (CAR) operably linked to a synthetic promoter, said promoter comprising two or more different TME dependent promoter response elements (PRE), wherein said at least two PRE's are an interferon-gamma-(IFN-γ) PRE and an NEκB PRE, and wherein the IFN-γ promoter response element comprises the nucleic acid sequence set forth in SEQ ID NO: 1, and wherein the NEκB PRE comprises the nucleic acid sequence set forth in SEQ ID NO: 2 ("K");
wherein the TME responsive expression vector is so constructed that
a) the presence of IFN-γ and TNFα in the TME, when binding to interferon-gamma-(IFN-γ) PRE and NEκB PRE, induces a higher expression level of CAR than when only IFN-γ or TNFα are present in the TME, and
b) minimal or low expression of CAR occurs in the absence of IFN-γ and TNFα,
so that the CAR is upregulated and directing activities against the tumor while sparing normal tissue.

2. The TME responsive expression vector of claim 1, further comprising a hypoxia PRE.

3. The TME responsive expression vector of any of claims 1-2, further comprising an externally inducible promoter and a trans-activator; wherein the synthetic promoter drives expression of the trans-activator and wherein the externally inducible promoter drives expression of the CAR; and wherein combined presence of the inducer and the TME factors induces expression of CAR.

4. The TME responsive expression vector of claim 3, wherein, in the presence of the external inducer and in the absence of TME factor, essentially no CAR expression is detected; preferably wherein, when the TME factor binds the promoter response element in the absence of the external inducer, essentially no CAR expression is detected.

5. The TME responsive expression vector of any of claims 3-4, wherein the externally inducible promoter is a Tet-Response-Element promoter and the external inducer is doxycycline and/or tetracycline, and wherein the Tet-Response-Element is activated by the combined presence of the trans-activator and doxycycline and/or tetracycline; preferably wherein the trans-activator is rtTA3.

6. The TME responsive expression vector of any of claims 1-5, wherein the CAR molecule encoded by the CAR sequence comprises an antigen binding domain, a transmembrane domain, and an intracellular domain comprising a costimulatory domain and/or a primary signaling domain, wherein said antigen binding domain binds to a disease associated tumor antigen.

7. The TME responsive expression vector of any of claims 1-6, wherein the vector is selected from a DNA vector, a plasmid, a lentivirus vector, an adenoviral vector, or a retrovirus vector.

8. An immune effector cell comprising the TME responsive expression vector of any of claims 1-7.

9. The immune effector cell of claim 8 for use as a medicament; or for treating a tumor of a patient in need thereof; preferably wherein the tumor is a solid tumor.

## Patentansprüche

1. Auf Tumormikromilieu (Tumor Micro Environment, TME) reagierender Expressionsvektor, umfassend:
eine Nukleinsäuresequenz, die einen chimären Antigen-Rezeptor (CAR) codiert, in operativer Verknüpfung mit einem synthetischen Promotor, wobei der Promotor zwei oder mehr unterschiedliche TMEabhängige Promotor-Response-Elemente (PRE) umfasst, wobei es sich bei den wenigstens zwei PRE um ein Interferon-gamma-(IFN-γ)-PRE und ein NFκB-PRE handelt und wobei das IFN-γ-Promotor-Response-Element die Nukleinsäuresequenz gemäß SEQ ID NO: 1 umfasst und wobei das NFκB-PRE die Nukleinsäuresequenz gemäß SEQ ID NO: 2 ("K") umfasst;
wobei der auf TME reagierende Expressionsvektor so konstruiert ist, dass
a) das Vorliegen von IFN-γ und TNFα im TME bei deren Bindung an Interferon-gamma-(IFN-γ)-PRE und NFκB-PRE ein höheres CAR-Expressionsniveau induziert als wenn nur IFN-γ oder TNFα im TME vorliegt und
b) minimale niedrige CAR-Expression in Abwesenheit von IFN-γ und TNFα erfolgt,
so dass der CAR heraufreguliert ist und Aktivitäten gegen den Tumor steuert, während Normalgewebe verschont wird.

2. Auf TME reagierender Expressionsvektor nach Anspruch 1, ferner umfassend ein Hypoxie-PRE.

3. Auf TME reagierender Expressionsvektor nach einem der Ansprüche 1-2, ferner umfassend einen extern induzierbaren Promotor und einen trans-Aktivator; wobei der synthetische Promotor die Expression des trans-Aktivators treibt und wobei der extern induzierbare Promotor den CAR treibt; und wobei ein gemeinsames Vorliegen des Induktors und der TME-Faktoren die CAR-Expression induziert.

4. Auf TME reagierender Expressionsvektor nach Anspruch 3, wobei in Gegenwart des externen Induktors und in Abwesenheit von TME-Faktor im Wesentlichen keine CAR-Expression nachgewiesen wird; vorzugsweise wobei, wenn der TME-Faktor das Promotor-Response-Element in Abwesenheit des externen Induktors bindet, im Wesentlichen keine CAR-Expression nachgewiesen wird.

5. Auf TME reagierender Expressionsvektor nach einem der Ansprüche 3-4, wobei es sich bei dem extern induzierbaren Promotor um einen Tet-Response-Element-Promotor und bei dem externen Induktor um Doxycyclin und/oder Tetracyclin handelt und wobei das Tet-Response-Element durch das gemeinsame Vorliegen des trans-Aktivators und von Doxycyclin und/oder Tetracyclin aktiviert wird; vorzugsweise wobei es sich bei dem trans-Aktivator um rtTA3 handelt.

6. Auf TME reagierender Expressionsvektor nach einem der Ansprüche 1-5, wobei das durch die CAR-Sequenz codierte CAR-Molekül eine Antigenbindungsdomäne, eine Transmembrandomäne und eine eine Costimulatordomäne und/oder eine primäre Signalgebungsdomäne umfassende intrazelluläre Domäne umfasst, wobei die Antigenbindungsdomäne an ein krankheitsassoziiertes Tumorantigen bindet.

7. Auf TME reagierender Expressionsvektor nach einem der Ansprüche 1-6, wobei der Vektor aus einem DNA-Vektor, einem Plasmid, einem Lentivirusvektor, einem Adenovirusvektor oder einem Retrovirusvektor ausgewählt ist.

8. Effektor-Immunzelle, umfassend den auf TME reagierenden Expressionsvektor nach einem der Ansprüche 1-7.

9. Effektor-Immunzelle nach Anspruch 8 zur Verwendung als Arzneimittel; oder zur Behandlung eines Tumors eines behandlungsbedürftigen Patienten; vorzugsweise wobei es sich bei dem Tumor um einen soliden Tumor handelt.

## Revendications

1. Vecteur d'expression réactif au micro-environnement tumoral (TME) comprenant :
une séquence d'acides nucléiques codant pour un récepteur antigénique chimérique (CAR) lié de manière fonctionnelle à un promoteur synthétique, ledit promoteur comprenant deux éléments de réponse du promoteur (PRE) dépendant du TME ou plus, lesdits au moins deux PRE étant un PRE à interférons-gamma-(IFN-γ) et un PRE NEκB, et l'élément de réponse du promoteur IFN-γ comprenant la séquence d'acides nucléiques indiquée dans la SEQ ID NO: 1, et le PRE NEκB comprenant la séquence d'acides nucléiques indiquée dans la SEQ ID NO: 2 (« K ») ;
le vecteur d'expression réactif au TME étant construit de telle manière que
a) la présence d'IFN-γ et TNFα dans le TME, lorsqu'il se lie à un PRE à interférons-gamma-(IFN-γ) et un PRE NFκB, induit un niveau d'expression supérieur de CAR que lorsque seulement IFN-γ ou TNFα est présent dans le TME, et
b) une expression minimale ou faible de CAR a lieu
en l'absence d'IFN-γ et TNFα,
de sorte que le CAR est régulé à la hausse et dirige des activités contre la tumeur tout en épargnant les tissus normaux.

2. Vecteur d'expression réactif au TME selon la revendication 1, comprenant en outre un PRE d'hypoxie.

3. Vecteur d'expression réactif au TME selon l'une quelconque des revendications 1 et 2, comprenant en outre un promoteur inductible de manière externe et un trans-activateur ; le promoteur synthétique entraînant l'expression du trans-activateur et le promoteur inductible de manière externe entraînant l'expression du CAR ; et une présence combinée de l'inducteur et des facteurs TME induisant une expression de CAR.

4. Vecteur d'expression réactif au TME selon la revendication 3, dans lequel, en la présence de l'inducteur externe et en l'absence du facteur TME, essentiellement aucune expression de CAR n'est détectée ; préférablement dans lequel, lorsque le facteur TME se lie à l'élément de réponse du promoteur en l'absence de l'inducteur externe, essentiellement aucune expression de CAR n'est détectée.

5. Vecteur d'expression réactif au TME selon l'une quelconque des revendications 3 et 4, le promoteur inductible de manière externe étant un promoteur d'élément de réponse du Tet et l'inducteur externe étant la doxycycline et/ou la tétracycline, et l'élément de réponse du Tet étant activé par la présence combinée du trans-activateur et de la doxycycline et/ou de la tétracycline ; préférablement, le trans-activateur étant rtTA3.

6. Vecteur d'expression réactif au TME selon l'une quelconque des revendications 1 à 5, la molécule de CAR codée par la séquence de CAR comprenant un domaine de liaison à un antigène, un domaine transmembranaire, et un domaine intracellulaire comprenant un domaine costimulatoire et/ou un domaine de signalisation primaire, ledit domaine de liaison à un antigène se liant à un antigène tumoral associé à une maladie.

7. Vecteur d'expression réactif au TME selon l'une quelconque des revendications 1 à 6, le vecteur étant choisi parmi un vecteur d'ADN, un plasmide, un vecteur de lentivirus, un vecteur adénoviral, ou un vecteur de rétrovirus.

8. Cellule effectrice immunitaire comprenant le vecteur d'expression réactif au TME selon l'une quelconque des revendications 1 à 7.

9. Cellule effectrice immunitaire selon la revendication 8 pour une utilisation en tant que médicament ; ou pour le traitement d'une tumeur d'un patient qui en a besoin ; préférablement, la tumeur étant une tumeur solide.
